# EUROPEAN PATENT APPLICATION

(11) **EP 2 163 564 A1**
(43) Date of publication of application: **17.03.2010**
(21) Application number: 09177410.9
(22) Date of filing: 21.01.2004
(51) Int. Cl.: C07K 16/30, G01N 33/574, A61K 39/395, A61P 35/00, C12N 5/18

(54) **Cancerous disease modifying antibodies**

(30) Priority: 21.01.2003 US 348231; 23.06.2003 US 603006; 19.12.2003 US 743451; 20.01.2004 US 762129
(62) Divisional of application: 04703738.7
(71) Applicant: F. Hoffmann-La Roche AG, 4070 Basel (CH)
(72) Inventor: Young, David S. F., Toronto Ontario M5J 2S7 (CA); Hahn, Susan E., Toronto Ontario M6P 3N7 (CA); Findlay, Helen P., Toronto Ontario M6P 3E9 (CA)
(74) Representative: Boakes, Jason Carrington

(57) **Abstract**

The present invention relates to a method for producing patient cancerous disease modifying antibodies using a novel paradigm of screening. By segregating the anti-cancer antibodies using cancer cell cytotoxicity as an end point, the process makes possible the production of anti-cancer antibodies for therapeutic and diagnostic purposes. The antibodies can be used in aid of staging and diagnosis of a cancer, and can be used to treat primary tumors and tumor metastases. The anti-cancer antibodies can be conjugated to toxins, enzymes, radioactive compounds, and hematogenous cells.

## Description

### FIELD OF THE INVENTION

This invention relates to the isolation and production of cancerous disease modifying antibodies (CDMAB) and to the use of these CDMAB in therapeutic and diagnostic processes, optionally in combination with one or more chemotherapeutic agents. The invention further relates to binding assays which utilize the CDMABs of the instant invention.

### BACKGROUND OF THE INVENTION

Each individual who presents with cancer is unique and has a cancer that is as different from other cancers as that person's identity. Despite this, current therapy treats all patients with the same type of cancer, at the same stage, in the same way. At least 30% of these patients will fail the first line therapy, thus leading to further rounds of treatment and the increased probability of treatment failure, metastases, and ultimately, death. A superior approach to treatment would be the customization of therapy for the particular individual. The only current therapy which lends itself to customization is surgery. Chemotherapy and radiation treatment can not be tailored to the patient, and surgery by itself, in most cases is inadequate for producing cures.

With the advent of monoclonal antibodies, the possibility of developing methods for customized therapy became more realistic since each antibody can be directed to a single epitope. Furthermore, it is possible to produce a combination of antibodies that are directed to the constellation of epitopes that uniquely define a particular individual's tumor.

Having recognized that a significant difference between cancerous and normal cells is that cancerous cells contain antigens that are specific to transformed cells, the scientific community has long held that monoclonal antibodies can be designed to specifically target transformed cells by binding specifically to these cancer antigens; thus giving rise to the belief that monoclonal antibodies can serve as "Magic Bullets" to eliminate cancer cells.

Monoclonal antibodies isolated in accordance with the teachings of the instantly disclosed invention have been shown to modify the cancerous disease process in a manner which is beneficial to the patient, for example by reducing the tumor burden, and will variously be referred to herein as cancerous disease modifying antibodies (CDMAB) or "anti-cancer" antibodies.

At the present time, the cancer patient usually has few options of treatment. The regimented approach to cancer therapy has produced improvements in global survival and morbidity rates. However, to the particular individual, these improved statistics do not necessarily correlate with an improvement in their personal situation.

Thus, if a methodology was put forth which enabled the practitioner to treat each tumor independently of other patients in the same cohort, this would permit the unique approach of tailoring therapy to just that one person. Such a course of therapy would, ideally, increase the rate of cures, and produce better outcomes, thereby satisfying a long-felt need.

Historically, the use of polyclonal antibodies has been used with limited success in the treatment of human cancers. Lymphomas and leukemias have been treated with human plasma, but there were few prolonged remission or responses. Furthermore, there was a lack of reproducibility and there was no additional benefit compared to chemotherapy. Solid tumors such as breast cancers, melanomas and renal cell carcinomas have also been treated with human blood, chimpanzee serum, human plasma and horse serum with correspondingly unpredictable and ineffective results.

There have been many clinical trials of monoclonal antibodies for solid tumors. In the 1980s there were at least four clinical trials for human breast cancer which produced only one responder from at least 47 patients using antibodies against specific antigens or based on tissue selectivity. It was not until 1998 that there was a successful clinical trial using a humanized anti-her 2 antibody in combination with Cisplatin. In this trial 37 patients were accessed for responses of which about a quarter had a partial response rate and another half had minor or stable disease progression.

The clinical trials investigating colorectal cancer involve antibodies against both glycoprotein and glycolipid targets. Antibodies such as 17-1A, which has some specificity for adenocarcinomas, had undergone Phase 2 clinical trials in over 60 patients with only one patient having a partial response. In other trials, use of 17-1A produced only one complete response and two minor responses among 52 patients in protocols using additional cyclophosphamide. Other trials involving 17-1A yielded results that were similar. The use of a humanized murine monoclonal antibody initially approved for imaging also did not produce tumor regression. To date there has not been an antibody that has been effective for colorectal cancer. Likewise there have been equally poor results for lung cancer, brain cancers, ovarian cancers, pancreatic cancer, prostate cancer, and stomach cancer. There has been some limited success in the use of anti-GD3 monoclonal antibody for melanoma. Thus, it can be seen that despite successful small animal studies that are a prerequisite for human clinical trials, the antibodies that have been tested have been for the most part ineffective.

### PRIOR PATENTS

U.S. Patent No. 5,750,102 discloses a process wherein cells from a patient's tumor are transfected with MHC genes which may be cloned from cells or tissue from the patient. These transfected cells are then used to vaccinate the patient.

U.S. Patent No. 4,861,581 discloses a process comprising the steps of obtaining monoclonal antibodies that are specific to an internal cellular component of neoplastic and normal cells of the mammal but not to external components, labeling the monoclonal antibody, contacting the labeled antibody with tissue of a mammal that has received therapy to kill neoplastic cells, and determining the effectiveness of therapy by measuring the binding of the labeled antibody to the internal cellular component of the degenerating neoplastic cells. In preparing antibodies directed to human intracellular antigens, the patentee recognizes that malignant cells represent a convenient source of such antigens.

U.S. Patent No.5,171,665 provides a novel antibody and method for its production. Specifically, the patent teaches formation of a monoclonal antibody which has the property of binding strongly to a protein antigen associated with human tumors, e.g. those of the colon and lung, while binding to normal cells to a much lesser degree.

U.S. Patent No. 5,484,596 provides a method of cancer therapy comprising surgically removing tumor tissue from a human cancer patient, treating the tumor tissue to obtain tumor cells, irradiating the tumor cells to be viable but non-tumorigenic, and using these cells to prepare a vaccine for the patient capable of inhibiting recurrence of the primary tumor while simultaneously inhibiting metastases. The patent teaches the development of monoclonal antibodies which are reactive with surface antigens of tumor cells. As set forth at col. 4, lines 45 et seq., the patentees utilize autochthonous tumor cells in the development of monoclonal antibodies expressing active specific immunotherapy in human neoplasia.

U.S. Patent No. 5,693,763 teaches a glycoprotein antigen characteristic of human carcinomas and not dependent upon the epithelial tissue of origin.

U.S. Patent No. 5,783,186 is drawn to Anti-Her2 antibodies which induce apoptosis in Her2 expressing cells, hybridoma cell lines producing the antibodies, methods of treating cancer using the antibodies and pharmaceutical compositions including said antibodies.

U.S. Patent No. 5,849,876 describes new hybridoma cell lines for the production of monoclonal antibodies to mucin antigens purified from tumor and non-tumor tissue sources.

U.S. Patent No. 5,869,268 is drawn to a method for generating a human lymphocyte producing an antibody specific to a desired antigen, a method for producing a monoclonal antibody, as well as monoclonal antibodies produced by the method. The patent is particularly drawn to the production of an anti-HD human monoclonal antibody useful for the diagnosis and treatment of cancers.

U.S. Patent No. 5,869,045 relates to antibodies, antibody fragments, antibody conjugates and single chain immunotoxins reactive with human carcinoma cells. The mechanism by which these antibodies function is two-fold, in that the molecules are reactive with cell membrane antigens present on the surface of human carcinomas, and further in that the antibodies have the ability to internalize within the carcinoma cells, subsequent to binding, making them especially useful for forming antibody-drug and antibody-toxin conjugates. In their unmodified form the antibodies also manifest cytotoxic properties at specific concentrations.

U.S. Patent No. 5,780,033 discloses the use of autoantibodies for tumor therapy and prophylaxis. However, this antibody is an antinuclear autoantibody from an aged mammal. In this case, the autoantibody is said to be one type of natural antibody found in the immune system. Because the autoantibody comes from "an aged mammal", there is no requirement that the autoantibody actually comes from the patient being treated. In addition the patent discloses natural and monoclonal antinuclear autoantibody from an aged mammal, and a hybridoma cell line producing a monoclonal antinuclear autoantibody.

### SUMMARY OF THE INVENTION

The instant inventors have previously been awarded U.S. Patent 6,180,357, entitled "Individualized Patient Specific Anti-Cancer Antibodies" directed to a process for selecting individually customized anti-cancer antibodies which are useful in treating a cancerous disease. For the purpose of this document, the terms "antibody" and "monoclonal antibody" (mAb) may be used interchangeably and refer to intact immunoglobulins produced by hybridomas (e.g. murine or human), immunoconjugates and, as appropriate, immunoglobulin fragments and recombinant proteins derived from immunoglobulins, such as chimeric and humanized immunoglobulins, F(ab') and F(ab')₂ fragments, single-chain antibodies, recombinant immunoglobulin variable regions (Fv)s, fusion proteins etc. It is well recognized in the art that some amino acid sequences can be varied in a polypeptide without significant effect on the structure or function of a protein. In the molecular rearrangement of antibodies, modifications in the nucleic or amino acid sequnce of the backbone region can generally be tolerated. These include, but are not limited to, substitutions (preferred are conservative substitutions), deletions or additions. Furthermore, it is within the purview of this invention to conjugate standard chemotherapeutic modalities, e.g, radionuclides, with the CDMAB of the instant invention, thereby focusing the use of said chemotherapeutics. The CDMAB can also be conjugated to toxins, cytotoxic moieties or enzymes e.g. biotin conjugated enzymes, or hematogenous cells.

This application utilizes the method for producing patient specific anti-cancer antibodies as taught in the '357 patent for isolating hybridoma cell lines which encode for cancerous disease modifying monoclonal antibodies. These antibodies can be made specifically for one tumor and thus make possible the customization of cancer therapy.
The prospect of individualized anti-cancer treatment will bring about a change in the way a patient is managed. A likely clinical scenario is that a tumor sample is obtained at the time of presentation, and banked. From this sample, the tumor can be typed from a panel of pre-existing cancerous disease modifying antibodies. The patient will be conventionally staged but the available antibodies can be of use in further staging the patient. The patient can be treated immediately with the existing antibodies, and a panel of antibodies specific to the tumor can be produced either using the methods outlined herein or through the use of phage display libraries in conjunction with the screening methods herein disclosed. All the antibodies generated will be added to the library of anti-cancer antibodies since there is a possibility that other tumors can bear some of the same epitopes as the one that is being treated. The antibodies produced according to this method may be useful to treat cancerous disease in any number of patients who have cancers that bind to these antibodies.

Using substantially the process of US Patent 6,180,357, the mouse monoclonal antibodies 7BD-33-11A, 1A245.6 and 11BD-2E11-2 were obtained following immunization of mice with cells from a patient's breast tumor biopsy. Within the context of this application, anti-cancer antibodies having either cell-killing (cytotoxic) or cell-growth inhibiting (cytostatic) properties will hereafter be referred to as cytotoxic. These antibodies can be used in aid of staging and diagnosis of a cancer, and can be used to treat tumor metastases. The 7BD-33-11A, 1A245.6 and 11BD-2E11-2 antigens were expressed on the cell surface of a broad range of human cell lines from different tissue origins. The breast cancer cell line MCF-7 and prostate cancer cell line PC-3 were the only 2 cancer cell lines tested that were susceptible to the cytotoxic effects of either 7BD-33-11A or 1A245.6. The breast cancer cell line MCF-7 and ovarian cancer cell line OVCAR-3 were the only two cancer cell lines tested that were susceptible to the cytotoxic effects of 11BD-2E11-2.

In addition to anti-cancer antibodies, the patient can elect to receive the currently recommended therapies as part of a multi-modal regimen of treatment. The fact that the antibodies isolated via the present methodology are relatively non-toxic to non-cancerous cells allows for combinations of antibodies at high doses to be used, either alone, or in conjunction with conventional therapy. The high therapeutic index will also permit re-treatment on a short time scale that should decrease the likelihood of emergence of treatment resistant cells.

The *in vitro* effects of 11BD-2E11-2 against breast and ovarian cancer cells were extended by establishing its anti-tumor activity *in vivo. In vivo* models of human cancer were established by implanting the MCF-7 breast cancer cells or OVCAR-3 ovarian cancer cells into severe combined immunodeficient (SCID) mice, as they are incapable of rejecting the human tumor cells due to a lack of certain immune cells. The effects of drugs tested in these kinds of pre-clinical xenograft tumor models are considered valid predictors of therapeutic efficacy. Cancer xenografts in mice grow as solid tumors developing parenchyma, stroma, central necrosis and neo-vasculature in the same manner as naturally occurring cancers. The mammary cancer cell line MCF-7 and the ovarian cancer cell line OVCAR-3 have been evaluated in SCID mice. The successful engraftment of both the MCF-7 and OVCAR-3 tumors and the sensitivity of the tumors to standard chemotherapeutic agents have characterized them as suitable models of human cancer for drug testing. The MCF-7 parental cell line and its variants and the OVCAR-3 cell line have been used successfully in xenograft tumor models to evaluate a wide range of therapeutic agents that have been used as clinical chemotherapeutic agents. 11BD-2E11-2 prevented tumor growth and reduced tumor burden in a preventative *in vivo* model of human breast cancer. Monitoring continued past 280 days post-treatment. 40 percent of the 11BD-2E11-2 treatment group was still alive at over 7.5 months post-implantation. Conversely, the isotype control group had 100 percent mortality after 6.5 months post-treatment. At day 51 (soon after last treatment), the mean tumor volume in the 11BD-2E11-2 treated group was 20% of the isotype control (p=0.0098). Therefore 11BD-2E11-2 enhanced survival and decreased the tumor burden compared to the control-treated groups in a well-established model of human breast cancer.

In addition to the beneficial effects in a model of human breast cancer, 11BD-2E11-2 treatment also had anti-tumor activity against OVCAR-3 cells in an ovarian cancer model. Body weight was used a surrogate measure of tumor progression in this model. At day 80 post-implantation (16 days after the end of treatment) the mice in the treated group had 87.6 percent the mean body weight of the control group (p=0.015). Thus, 11BD-2E11-2 treatment was efficacious as it delayed tumor progression compared to the buffer control treated group in a well-established model of human ovarian cancer. The anti-tumor activities of 11BD-2E11-2, in several different cancer models, make it an attractive anti-cancer therapeutic agent.

This invention teaches the use of the 11BD-2E11-2 antigen as a target for a therapeutic agent, that when administered can reduce the tumor burden of a cancer expressing the antigen in a mammal, and can also lead to a prolonged survival of the treated mammal. This invention also teaches the use of CDMAB (11BD-2E11-2), and its derivatives, to target its antigen to reduce the tumor burden of a cancer expressing the antigen in a mammal, and to prolong the survival of a mammal bearing tumors that express this antigen.

The result of 7BD-33-11A and 1A245.6 cytotoxicity against breast and prostate cancer cells in culture was further extended by establishing its anti-tumor activity *in vivo.* In an *in vivo* model of human cancer, the MB-231 breast cancer cells or PC-3 prostate cancer cells were implanted underneath the skin at the scruff of the neck of severe combined immunodeficient (SCID) mice, as they are incapable of rejecting the human tumor cells due to a lack of certain immune cells. Pre-clinical xenograft tumor models are considered valid predictors of therapeutic efficacy. Xenografts in mice grow as solid tumors developing stroma, central necrosis and neo-vasculature in the same manner as naturally occurring cancers. The mammary tumor cell line MB-231 and the prostate tumor cell line PC-3 have been evaluated as an *in vivo* xenograft model in immunodeficient mice. The successful engraftment or 'take-rate' of both the MB-231 and PC-3 tumors and the sensitivity of the tumors to standard chemotherapeutic agents have characterized them as suitable models. The MB-231 parental cell line and variants of the cell line and the PC-3 androgen-independent cell line have been used successfully in xenograft tumor models to evaluate a wide range of therapeutic agents that used as clinical chemotherapeutic agents.

Also, 7BD-33-11A and 1A245.6 prevented tumor growth and reduced tumor burden in a preventative *in vivo* model of human breast cancer. Monitoring continued past 150 days post-treatment. 7BD-33-11A never developed tumors and 87.5 percent of the 7BD-33-11A treatment group was still alive at over 6 months post-implantation. Conversely, the isotype control group had 100 percent mortality by day 72 (23 days post-treatment). 1A245.6 treated mice reached 100 percent mortality by day 151 post-treatment, which is greater than 6 times longer than the isotype control treatment group. Therefore 1A245.6, and to a greater extent 7BD-33-11A, enhanced survival and decreased the tumor burden in a breast cancer model.

Additionally, both 7BD-33-11A and 1A245.6 significantly suppressed tumor growth and decreased tumor burden in an established *in vivo* model of human breast cancer. By day 80 (23 days post-treatment), 7BD-33-11A treated mice had 83 percent lower mean tumor volumes in comparison to isotype control group (p=.001). 1A245.6 treatment also produced lower mean tumor volumes on this day, 35 percent (p=.135). Using survival as a measure of antibody efficacy, it was estimated that the risk of dying in the 7BD-33-11A treatment group was about 16 percent of the isotype control group (p=0.0006) at around 60 days post-treatment. 100 percent of the isotype control group died by 50 days post-treatment. In comparison, 1A245.6 treated mice survived until 100 days post-treatment and 60% of the 7BD-33-11A treatment groups were still alive at 130 days post-treatment. This data demonstrate that both 1A245.6 and 7BD-33-11A treatments conferred a survival and reduced tumor burden benefit compared to the control treated group. 7BD-33-11A and 1A245.6 treatment appeared safe, as it did not induce any signs of toxicity, including reduced body weight and clinical distress. Thus, 7BD-33-11A and 1A245.6 treatment was efficacious as it both delayed tumor growth and enhanced survival compared to the control-treated groups in a well-established model of human breast cancer.

In addition to the beneficial effects in the established *in vivo* tumor model of breast cancer, 7BD-33-11A and 1A245.6 treatment also had anti-tumor activity against PC-3 cells in a preventative *in vivo* prostate cancer model. In this prostate xenograft model, 7BD-33-11A and 1A245.6 were given separately to mice 1 day prior to implantation of tumor cells followed by weekly injections for 7 weeks. 7BD-33-11A and 1A245.6 treatment was significantly (p=.001 and .017 respectively) more effective in suppressing tumor growth shortly after the treatment period than an isotype control antibody. At the end of the treatment phase, mice given 7BD-33-11A or 1A245.6 had tumors that grew to only 31 and 50 percent of the isotype control group respectively.

For PC-3 SCID xenograft models, body weight can be used as a surrogate indicator of disease progression. On day 52, both 7BD-33-11A and 1A245.6 treatment significantly (p=.002 and .004 respectively) prevented the loss of body weight by 54 and 25 percent respectively in comparison to isotype control. Mice were monitored for survival post-treatment. At 11 days post-treatment, isotype and buffer control mice had reached 100 percent mortality. Conversely, 7BD-33-11A and 1A245.6 reached 100 percent mortality at day 38 post-treatment, 3 times longer than the control groups. Thus, 7BD-33-11A and 1A245.6 treatment was efficacious as it both delayed tumor growth, prevented body weight loss and extended survival compared to the isotype control treated group in a well-established model of human prostate cancer.

In addition to the preventative *in vivo* tumor model of prostate cancer, 7BD-33-11A demonstrated anti-tumor activity against PC-3 cells in an established *in vivo* tumor model. In this xenograft model, PC-3 prostate cancer cells were transplanted subcutaneously into SCID mice such that the tumor reached a certain size before antibody treatment. Treatment with 7BD-33-11A was again compared to isotype control. It was shown that the 7BD-33-11A treatment group had significantly (p<0.024) smaller mean tumor volumes compared with the isotype control treated group immediately following treatment. 7BD-33-11A treatment mediated tumor suppression by 36 percent compared to the isotype control group. The anti-tumor activities of 7BD-33-11A, in several different cancer models, make it an attractive anti-cancer therapeutic agent.

The binding of 7BD-33-11A and 1A245.6 towards normal human tissues was determined. By IHC staining, the majority of the tissues failed to express the 7BD-33-11A antigen, including the vital organs, such as the kidney, heart, and lung. 7BD-33-11A stained the salivary gland, liver, pancreas, stomach, prostate and duodendum, and strongly stained the tonsil. Results from tissue staining indicated that 7BD-33-11A showed restricted binding to various cell types but had binding to infiltrating macrophages, lymphocytes, and fibroblasts. For 1A245.6, a wider range of tissues was positively stained. For the majority of cases, staining was restricted to the epithelium or infiltrating macrophages, lymphocytes, and fibroblasts. However, positive staining was seen on both cardiac muscle and hepatocytes. 7BD-33-11A and 1A245.6 displayed both membrane and cytoplasmic staining patterns.

Localization of the 7BD-33-11A and 1A245.6 antigen and its prevalence within breast cancer patients is important in assessing the benefits of immunotherapy. To address antigen expression in breast tumors from cancer patients, tumor tissue samples from 50 individual breast cancer patients were screened for expression of either the 7BD-33-11A or 1A245.6 antigen. The results of the study showed that 36 percent of tissue samples positively stained for the 7BD-33-11A antigen. Expression of 7BD-33-11A within patient samples appeared specific for cancer cells as staining was restricted to malignant cells. In addition, 7BD-33-11A stained 0 of 10 samples of normal tissue from breast cancer patients. On the other hand, 1A245.6 stained 98 percent of breast cancer tissue samples. 1A245.6 also stained 8 out of 10 samples of normal tissue from breast cancer patients. However, in general this staining was much weaker than that observed with the breast cancer tissue samples and was generally restricted to infiltrating fibroblasts. 7BD-33-11A and 1A245.6 expression was further evaluated based on breast tumor expression of the receptors for the hormones estrogen and progesterone, which play an important role in the development, treatment, and prognosis of breast tumors. No correlation was apparent between expression of the 1A245.6 antigen and expression of the receptors for either estrogen or progesterone. There was a slight correlation between estrogen or progesterone receptor expression and expression of 7BD-33-11A; tissues with receptor expression had slightly higher 7BD-33-11A expression. When tumors were analyzed based on their stage, or degree to which the cancer advanced, results suggested a trend towards greater positive expression with higher tumor stage for 7BD-33-11A and higher intensity staining with higher tumor stage for 1A245.6. However, the results were limited by the small sample size.

To further extend the potential therapeutic benefit of 7BD-33-11A and 1A245.6, the frequency and localization of the antigen within various human cancer tissues was determined. Several cancer types, in addition to breast cancer, expressed the 7BD-33-11A antigen. The positive human cancer types included skin (1/2), lung (3/4), liver (2/3), stomach (4/5), thyroid (2/2), prostate (1/1), uterus (4/4) and kidney (3/3). Some cancers did not express the antigen; these included ovary (0/3), testis (0/1), brain (0/2) and lymph node (0/2). For 1A245.6, as with the normal human tissue array, a multitude of cancers from various human tissue types were positively stained. Greater staining was seen on malignant cells of the skin, lung, liver, uterus, kidney, stomach and bladder. As with human breast cancer tissue, localization of 7BD-33-11A and 1A245.6 occurred both on the membrane and within the cytoplasm of these tumor cells. So, in addition to the 7BD-33-11A and 1A245.6 antibody binding to cancer cell lines *in vitro,* there is evidence that the antigen is expressed in humans, and on multiple types of cancers.

*In toto,* this data demonstrates that both the 7BD-33-11A and 1A245.6 antigen is a cancer associated antigen and is expressed in humans, and is a pathologically relevant cancer target. Further, this data also demonstrates the binding of 7BD-33-11A and 1A245.6 antibody to human cancer tissues, and can be used appropriately for assays that can be diagnostic, predictive of therapy, or prognostic. In addition, the cell membrane localization of this antigen permits the use of this antigen, its gene or derivatives, its protein or its variants to be used for assays that can be diagnostic, predictive of therapy, or prognostic.

In all, this invention teaches the use of the 7BD-33-11A or 1A245.6 antigen as a target for a therapeutic agent, that when administered can reduce the tumor burden of a cancer expressing the antigen in a mammal, and can also lead to a prolonged survival of the treated mammal. This invention also teaches the use of CDMAB (7BD-33-11A/1A245.6), and its derivatives, to target its antigen to reduce the tumor burden of a cancer expressing the antigen in a mammal, and to prolong the survival of a mammal bearing tumors that express this antigen. Furthermore, this invention also teaches the use of detecting the 7BD-33-11A or 1A245.6 antigen in cancerous cells that can be useful for the diagnosis, prediction of therapy, and prognosis of mammals bearing tumors that express this antigen.

If a patient is refractory to the initial course of therapy or metastases develop, the process of generating specific antibodies to the tumor can be repeated for re-treatment. Furthermore, the anti-cancer antibodies can be conjugated to red blood cells obtained from that patient and re-infused for treatment of metastases. There have been few effective treatments for metastatic cancer and metastases usually portend a poor outcome resulting in death. However, metastatic cancers are usually well vascularized and the delivery of anti-cancer antibodies by red blood cells can have the effect of concentrating the antibodies at the site of the tumor. Even prior to metastases, most cancer cells are dependent on the host's blood supply for their survival and anti-cancer antibody conjugated to red blood cells can be effective against *in situ* tumors as well. Alternatively, the antibodies may be conjugated to other hematogenous cells, e.g. lymphocytes, macrophages, monocytes, natural killer cells, etc.

There are five classes of antibodies and each is associated with a function that is conferred by its heavy chain. It is generally thought that cancer cell killing by naked antibodies are mediated either through antibody dependent cellular cytotoxicity or complement dependent cytotoxicity. For example murine IgM and IgG2a antibodies can activate human complement by binding the C-1 component of the complement system thereby activating the classical pathway of complement activation which can lead to tumor lysis. For human antibodies the most effective complement activating antibodies are generally IgM and IgG1. Murine antibodies of the IgG2a and IgG3 isotype are effective at recruiting cytotoxic cells that have Fc receptors which will lead to cell killing by monocytes, macrophages, granulocytes and certain lymphocytes. Human antibodies of both the IgGl and IgG3 isotype mediate ADCC.

Another possible mechanism of antibody mediated cancer killing may be through the use of antibodies that function to catalyze the hydrolysis of various chemical bonds in the cell membrane and its associated glycoproteins or glycolipids, so-called catalytic antibodies.

There are two additional mechanisms of antibody mediated cancer cell killing which are more widely accepted. The first is the use of antibodies as a vaccine to induce the body to produce an immune response against the putative cancer antigen that resides on the tumor cell. The second is the use of antibodies to target growth receptors and interfere with their function or to down regulate that receptor so that effectively its function is lost.

The clinical utility of a cancer drug is based on the benefit of the drug under an acceptable risk profile to the patient. In cancer therapy survival has generally been the most sought after benefit, however there are a number of other well-recognized benefits in addition to prolonging life. These other benefits, where treatment does not adversely affect survival, include symptom palliation, protection against adverse events, prolongation in time to recurrence or disease-free survival, and prolongation in time to progression. These criteria are generally accepted and regulatory bodies such as the U.S. Food and Drug Administration (F.D.A.) approve drugs that produce these benefits (Hirschfeld et al. Critical Reviews in Oncology/Hematolgy 42:137-143 2002). In addition to these criteria it is well-recognized that there are other endpoints that may presage these types of benefits. In part, the accelerated approval process granted by the U.S. F.D.A. acknowledges that there are surrogates that will likely predict patient benefit. As of year-end (2003), there has been sixteen drugs approved under this process, and of these, four have gone on to full approval, i.e., follow-up studies have demonstrated direct patient benefit as predicted by surrogate endpoints. One important endpoint for determining drug effects in solid tumors is the assessment of tumor burden by measuring response to treatment (Therasse et al. Journal of the National Cancer Institute 92(3):205-216 2000). The clinical criteria (RECIST criteria) for such evaluation have been promulgated by Response Evaluation Criteria in Solid Tumors Working Group, a group of international experts in cancer. Drugs with a demonstrated effect on tumor burden, as shown by objective responses according to RECIST criteria, in comparison to the appropriate control group tend to, ultimately, produce direct patient benefit. In the pre-clinical setting tumor burden is generally more straightforward to assess and document. In that pre-clinical studies can be translated to the clinical setting, drugs that produce prolonged survival in pre-clinical models have the greatest anticipated clinical utility. Analogous to producing positive responses to clinical treatment, drugs that reduce tumor burden in the pre-clinical setting may also have significant direct impact on the disease. Although prolongation of survival is the most sought after clinical outcome from cancer drug treatment, there are other benefits that have clinical utility and it is clear that tumor burden reduction can also lead to direct benefits and have clinical impact (Eckhardt et al. Developmental Therapeutics: Successes and Failures of Clinical Trial Designs of Targeted Compounds; ASCO Educational Book, 39th Annual Meeting, 2003, pages 209-219).

Accordingly, it is an objective of the invention to utilize a method for producing cancerous disease modifying antibodies from cells derived from a particular individual which are cytotoxic with respect to cancer cells while simultaneously being relatively non-toxic to non-cancerous cells, in order to isolate hybridoma cell lines and the corresponding isolated monoclonal antibodies and antigen binding fragments thereof for which said hybridoma cell lines are encoded.

It is an additional objective of the invention to teach cancerous disease modifying antibodies and antigen binding fragments thereof.

It is a further objective of the instant invention to produce cancerous disease modifying antibodies whose cytotoxicity is mediated through antibody dependent cellular toxicity.

It is yet an additional objective of the instant invention to produce cancerous disease modifying antibodies whose cytotoxicity is mediated through complement dependent cellular toxicity.

It is still a further objective of the instant invention to produce cancerous disease modifying antibodies whose cytotoxicity is a function of their ability to catalyze hydrolysis of cellular chemical bonds.

A still further objective of the instant invention is to produce cancerous disease modifying antibodies which are useful for in a binding assay for diagnosis, prognosis, and monitoring of cancer.

Other objects and advantages of this invention will become apparent from the following description wherein are set forth, by way of illustration and example, certain embodiments of this invention.

### BRIEF DESCRIPTION OF THE FIGURES

This patent or application file contains at least one drawing executed in color. Copies of this patent or patent application publication with color drawing(s) will be provided by the Office upon request and payment of the necessary fee.
Figure 1 includes representative FACS histograms of 1A245.6 antibodies, isotype control antibodies for both antibodies, anti-EGFR antibodies directed against several cancer cell lines and non-cancer cells.
Figure 2 includes representative FACS histograms of 7BD-33-11A antibodies, isotype control antibodies for 1A245.6, anti-EGFR antibodies, isotype control antibodies for anti-EGFR directed against several cancer cell lines and non-cancer cells.
Figure 3 includes representative FACS histograms of 11BD-2E11-2 antibodies, isotype control antibodies for both antibodies, anti-EGFR antibodies directed against several cancer cell lines and non-cancer cells.
Figure 4 is a graphical analysis of tumor volume over time with respect to particular antibody treatment.
Figure 5 is a graphical analysis of antibody effect on MB231 Human Breast Cancer tumor volume over time.
Figure 6 is a graphical analysis quantifying percent survival over time relative to antibody therapy.
Figure 7. Effect of 11BD-2E11-2 on tumor growth in a preventative MCF-7 breast cancer model. The dashed line indicates the period during which the antibody was administered. Data points represent the mean +/- SEM.
Figure 8. Survival of tumor-bearing mice after treatment with 11BD-2E11-2 or isotype control antibody in a preventative MCF-7 xenograft study. Mice were monitored for survival for longer than 230 days post-treatment.
Figure 9. Effect of 11BD-2E11-2 on mean body weight in a preventative OVCAR-3 ovarian cancer model. The solid line indicates the period during which the antibody was administered. Data points represent the mean +/- SEM.
Figure 10. Survival of tumor-bearing mice after treatment with 11BD-2E11-2 or buffer control antibody in a preventative OVCAR-3 study. Mice were monitored for survival for approximately 60 days post-treatment.
Figure 11. Survival of tumor-bearing mice after treatment with 7BD-33-11A, 1A245.6 or isotype control antibody in a preventative MB-231 xenograft study. Mice were monitored for survival for 200 days post-treatment.
Figure 12. Effect of 7BD-33-11A and 1A245.6 on tumor growth in a preventative MB-231 breast cancer model. The dashed line indicates the period during which the antibody was administered. Data points represent the mean +/- SEM.
Figure 13. Survival of tumor-bearing mice after treatment with 7BD-33-11A, 1A245.6 or isotype control antibody in an established MB-231 xenograft study. Mice were monitored for survival for 130 days post-treatment.
Figure 14. Effect of 7BD-33-11A and 1A245.6 on tumor growth in a preventative PC-3 prostate cancer model. The dashed line indicates the period during which the antibody was administered. Data points represent the mean +/- SEM.
Figure 15. Histogram showing mean body weight of the different treatment groups over the duration of the preventative PC-3 xenograft study. Data are presented as the mean +/- SEM for each group at each time point.
Figure 16. Survival of tumor-bearing mice after treatment with 7BD-33-11A, 1A245.6, isotype or buffer control antibody in a preventative PC-3 xenograft study. Mice were monitored for survival for 38 days post-treatment.
Figure 17. Effect of 7BD-33-11A and 1A245.6 on tumor growth in an established PC-3 prostate cancer model. The dashed line indicates the period during which the antibody was administered. Data points represent the mean +/- SEM.
Figure 18. Histogram showing mean body weight of the different treatment groups over the duration of the established PC-3 xenograft study. Data are presented as the mean +/- SEM for each group at each time point.
Figures 19A-C. Normal Human Brain A. 7BD-33-11A. B. 1A245.6. C. Negative isotype control. Magnification is 200X.
Figures 20A-C Normal Human Heart A. 7BD-33-11A. B. 1A245.6 (arrows indicate positive staining). C. Negative isotype control. Magnification is 200X.
Figures 21A-C. Normal Human Stomach Antrum. A. 7BD-33-11A (arrows indicate positive staining of gastric gland epithelium). B. 1A245.6 (arrows indicate positive staining of gastric gland epithelium). C. Negative isotype control. Magnification is 200X.
Figures 22A-B. Representative micrograph of 7BD-33-11 A binding to human breast cancer tumor (infiltrating duct carcinoma; Panel A; black arrows: sheets of tumor cells, yellow arrow: tumor stroma) and human normal breast (Panel B). Magnification is 200X.
Figures 23A-B. Representative micrograph of 1A245.6 binding to human breast cancer tumor (infiltrating duct carcinoma; Panel A; black arrows: sheets of tumor cells, yellow arrow: tumor stroma) and human normal breast (Panel B; black arrows: fibroblasts). Magnification is 200X.
Figures 24A-C. Renal Cell Carcinoma. A. 7BD-33-11A (arrows indicate positive staining in sheets of tumor cells). B. 1A245.6 (arrows indicate positive staining in sheets of tumor cells). C. Negative isotype control. Magnification is 200X.

### EXAMPLE 1

### Hybridomas Production - Hybridoma Cell Line_7BD-33-11A, 1A245.6, 11BD-2E11-2 Hybridomas:

The hybridoma cell lines 7BD-33-11A and 1A245.6 were deposited, in accordance with the Budapest Treaty, with the American Type Culture Collection, 10801 University Blvd., Manassas, VA 20110-2209 on January 8, 2003, under Accession Number PTA-4890 and PTA-4889, respectively. In accordance with 37 CFR 1.808, the depositors assure that all restrictions imposed on the availability to the public of the deposited materials will be irrevocably removed upon the granting of a patent.

The hybridoma cell line 11BD-2E11-2 was deposited, in accordance with the Budapest Treaty, with the American Type Culture Collection, 10801 University Blvd., Manassas, VA 20110-2209 on November 11, 2003, under Accession Number PTA-5643. In accordance with CFR 1.808, the depositors assure that all restrictions imposed on the availability to the public of the deposited materials will be irrevocably removed upon the granting of a patent.

To produce the hybridoma that produce the anti-cancer antibody 7BD-33-11A single cell suspensions of the antigen, i.e. human breast cancer cells, were prepared in cold PBS. Eight to nine weeks old BALB/c mice were immunized by injecting 100 microliters of the antigen-adjuvant containing between 0.2 million and 2.5 million cells in divided doses both subcutaneously and intraperitoneally with Freund's Complete Adjuvant. Freshly prepared antigen-adjuvant was used to boost the immunized mice at between 0.2 million and 2.5 million cells in the same fashion three weeks after the initial immunization, and two weeks after the last boost. A spleen was used for fusion at least two days after the last immunization. The hybridomas were prepared by fusing the isolated splenocytes with Sp2/0 myeloma partners. The supernatants from the fusions were tested for subcloning of the hybridomas.

To produce the hybridoma that produce the anti-cancer antibody 1A245.6 single cell suspensions of the antigen, i.e. human breast cancer cells, were prepared in cold PBS. Eight to nine weeks old BALB/c mice were immunized by injecting 100 microliters of the antigen-adjuvant containing 2.5 million cells in divided doses both subcutaneously and intraperitoneally with Freund's Complete Adjuvant. Freshly prepared antigen-adjuvant was used to boost the immunized mice at 2.5 million cells in the same fashion three weeks after the initial immunization, two weeks later, five weeks later and three weeks after the last boost. A spleen was used for fusion at least three days after the last immunization. The hybridomas were prepared by fusing the isolated splenocytes with NSO-1 myeloma partners. The supernatants from the fusions were tested for subcloning of the hybridomas.

To produce the hybridoma that produce the anti-cancer antibody 11BD-2E11-2 single cell suspensions of the antigen, i.e. human breast cancer cells, were prepared in cold PBS. Eight to nine weeks old BALB/c mice were immunized by injecting 100 microliters of the antigen-adjuvant containing between 0.2 million and 2.5 million cells in divided doses both subcutaneously and innaperitoneally with Freund's Complete Adjuvant. Freshly prepared antigen-adjuvant was used to boost the immunized mice at between 0.2 million and 2.5 million cells in the same fashion two to three weeks after the initial immunization, and two weeks after the last boost. A spleen was used for fusion at least two days after the last immunization. The hybridomas were prepared by fusing the isolated splenocytes with NSO-1 myeloma partners. The supernatants from the fusions were tested for subcloning of the hybridomas.

To determine whether the antibodies secreted by hybridoma cells are of the IgG or IgM isotype, an ELISA assay was employed. 100 microliters/well of goat anti-mouse IgG + IgM (H+L) at a concentration of 2.4 micrograms/mL in coating buffer (0.1M carbonate/bicarbonate buffer, pH 9.2-9.6) at 4°C was added to the ELISA plates overnight. The plates were washed thrice in washing buffer (PBS + 0.05% Tween). 100 microliters/well blocking buffer (5% milk in wash buffer) was added to the plate for 1 hr. at room temperature and then washed thrice in washing buffer. 100 microliters/well of hybridoma supernatant was added and the plate incubated for 1 hr. at room temperature. The plates were washed thrice with washing buffer and 1/5000 dilution of either goat anti-mouse IgG or IgM horseradish peroxidase conjugate (diluted in PBS containing 1% bovine serum albumin), 100 microliters/well, was added. After incubating the plate for 1 hr. at room temperature the plate was washed thrice with washing buffer. 100 microliters/well of TMB solution was incubated for 1-3 minutes at room temperature. The color reaction was terminated by adding 100 microliters/well 2M H₂SO₄ and the plate was read at 450 nm with a Perkin-Elmer HTS7000 plate reader. As indicated in Table 1 the 7BD-33-11A, 1A245.6, 11BD-2E11-2 hybridomas secreted primarily antibodies of the IgG isotype.

After one to four rounds of limiting dilution hybridoma supernatants were tested for antibodies that bound to target cells in a cell ELISA assay. Three breast cancer cell lines were tested: MDA-MB-231 (also referred to as MB-231), MDA-MB-468 (also referred to as MB-468), and SKBR-3. The plated cells were fixed prior to use. The plates were washed thrice with PBS containing MgCl₂ and CaCl₂ at room temperature. 100 microliters of 2% paraformaldehyde diluted in PBS was added to each well for ten minutes at room temperature and then discarded. The plates were again washed with PBS containing MgCl₂ and CaCl₂ three times at room temperature.. Blocking was done with 100 microliters/well of 5% milk in wash buffer (PBS + 0.05% Tween) for 1 hr at room temperature. The plates were washed thrice with wash buffer and the hybridoma supernatant was added at 100 microliters/well for 1 hr at room temperature. The plates were washed three times with wash buffer and 100 microliters/well of 1/5000 dilution of goat anti-mouse IgG or IgM antibody conjugated to horseradish peroxidase (diluted in PBS containing 1% bovine serum albumin) was added. After a one hour incubation at room temperature the plates were washed three times with wash buffer and 100 microliter/well of TMB substrate was incubated for 1-3 minutes at room temperature. The reaction was terminated with 100 microliters/well 2M H₂SO₄ and the plate read at 450 nm with a Perkin-Elmer HTS7000 plate reader. The results as tabulated in Table 1 were expressed as the number of folds above background compared to the IgG isotype control (3BD-27). The antibodies from the 7BD-33-11A and 1A245.6 hybridoma cell lines bound strongly to all 3 breast lines, with binding at least 6 times greater than background. Both antibodies bound most strongly to the MDA-MB-231 cell line. The antibodies from the 11BD-2E11-2 hybridoma cell line also bound most strongly to the MDA-MB-231 cell line, but did not demonstrate binding on the other 2 cell lines greater than background. These results suggest that the epitope recognized by this antibody is not present on MDA-MB-468 or SKBR-3 cells, and is distinct from the epitopes recognized by 7BD-33-11A and 1A245.6.

In conjunction with testing for antibody binding the cytotoxic effect of the hybridoma supernatants were tested in the same breast cancer cell lines: MDA-MB-231, MDA-MB-468 and SKBR-3. The Live/Dead cytotoxicity assay was obtained from Molecular Probes (Eu,OR). The assays were performed according to the manufacturer's instructions with the changes outlined below. Cells were plated before the assay at the predetermined appropriate density. After 2 days, 100 microliters of supernatant from the hybridoma microtitre plates were transferred to the cell plates and incubated in a 5% CO₂ incubator for 5 days. The wells that served as the positive controls were aspirated until empty and 100 microliters of sodium azide and/or cycloheximide was added. 3BD-27 monoclonal antibody was also added as an isotype control since it was known not to bind to the three breast cancer cell lines being tested. An anti-EGFR antibody (C225) was also used in the assay for comparison. After 5 days of treatment, the plate was then emptied by inverting and blotted dry. Room temperature DPBS containing MgCl₂ and CaCl₂ was dispensed into each well from a multichannel squeeze bottle, tapped three times, emptied by inversion and then blotted dry. 50 microliters of the fluorescent Live/Dead dye diluted in DPBS containing MgCl₂ and CaCl₂ was added to each well and incubated at 37°C in a 5% CO₂ incubator for 30 minutes. The plates were read in a Perkin-Elmer HTS7000 fluorescence plate reader and the data was analyzed in Microsoft Excel. The results were tabulated in Table 1.

Differential cytotoxicity was observed with the 3 antibodies. 11BD-2E11-2 demonstrated killing of 39-73%, with the highest cytotoxicity observed in SKBR-3 cells. 1A245.6 and 7BD-33-11A demonstrated similar cytotoxicity in MDA-MB-231 cells, but 1A245.6 was also cytotoxic to MDA-MB-468 cells, while 7BD-33-11A was not.

This indicated the antibody derived form the hybridoma cell can produce cytotoxicity in cancer cells. There was also a general association between the degree of antibody binding and the cytotoxicity produced by the hybridoma supernatants. There were several exceptions to this trend such as the amount of cytotoxicity produced by 11BD-2E11-2 in MB-468 cancer cells, and SKBR-3 cancers despite a paucity of binding. This suggested that the antibody has a mediating action that was not detected by the cell ELISA binding assay in this cell type, or the assay did not detect the binding, which may be due to the constraints of the assay such as cell fixation. Finally, there existed yet another possibility, that is, the assay was not sensitive enough to detect the binding that was sufficient to mediate cytotoxicity in this particular situation. The other exception was the relative paucity of cytotoxicity of 7BD-33-11 A towards MB-468 cells despite a 6 fold increase in binding over the background in comparison to an isotype control. This pointed to the possibility that binding was not necessarily predictive of the outcome of antibody ligation of its cognate antigen. The known nonspecific cytotoxic agents cycloheximide produced cytotoxicity as expected.

| Table 1 | **Cytotoxicity(%)** | | | | | | **Binding (above bkgd)** | | |
|---|---|---|---|---|---|---|---|---|---|
| | **MB-231** | | **MB-468** | | **SKBR-3** | | **MB-231** | **MB-468** | **SKBR-3** |
| Clone | Average | CV | Average | CV | Average | CV | Fold | Fold | Fold |
| 1A245.6 | 17 | 7 | 13 | 5 | 44 | 8 | 23 | 10 | 16 |
| 7BD-33-11A | 16 | 2 | 2 | 2 | 29 | 3 | 13 | 6 | 9 |
| 11BD-2E11-2 | 39 | 2 | 66 | 1 | 73 | 18 | 11 | 2 | 1 |
| Cyclcheximide | 49 | 9 | 24 | 5 | 56 | 14 | | | |

### EXAMPLE 2

### Antibody Production

Monoclonal antibodies were produced by culturing the hybridomas, 7BD-33-11A, 1A245.6, 11BD-2E11-2, in CL-1000 flasks (BD Biosciences, Oakville, ON) with collections and reseeding occurring twice/week and standard antibody purification procedures with Protein G Sepharose 4 Fast Flow (Amersham Biosciences, Baie d'Urfé, QC). It is within the scope of this invention to utilize monoclonal antibodies which are humanized, chimerized or murine antibodies. 7BD-33-11A, 1A245.6, 11BD-2E11-2 were compared to a number of both positive (anti-Fas (EOS9.1, IgM, kappa, 20 micrograms/mL, eBioscience, San Diego, CA), anti-Her2/neu (IgGl, kappa, 10 microgram/mL, Inter Medico, Markham, ON), anti-EGFR (C225, IgG1, kappa, 5 microgram/mL, Cedarlane, Homby, ON), Cycloheximide (100 micromolar, Sigma, Oakville, ON), NaN₃ (0.1%, Sigma, Oakville, ON)) and negative (107.3 (anti-TNP, IgGl, kappa, 20 microgram/mL, BD Biosciences, Oakville, ON), G155-178 (anti-TNP, IgG2a, kappa, 20 microgram/mL, BD Biosciences, Oakville, ON), MPC-11 (antigenic specificity unknown, IgG2b, kappa, 20 microgram/mL), J606 (anti-fructosan, IgG3, kappa, 20 microgram/mL), IgG Buffer (2%)) controls in a cytotoxicity assay (Table 2).

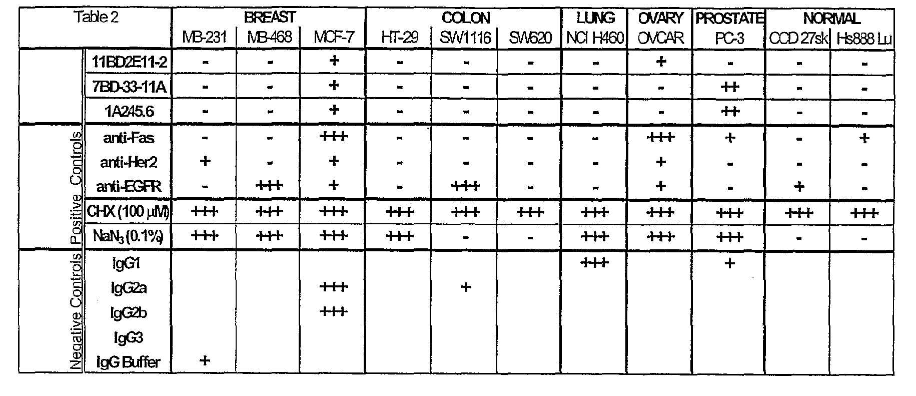

Breast cancer (MB-231, MB-468, MCF-7), colon cancer (HT-29, SW1116, SW620), lung cancer (NCI H460), ovarian cancer (OVCAR), prostate cancer (PC-3), and non-cancer (CCD 27sk, Hs888 Lu) cell lines were tested (all from the ATCC, Manassas, VA). The Live/Dead cytotoxicity assay was obtained from Molecular Probes (Eugene,OR). The assays were performed according to the manufacturer's instructions with the changes outlined below. Cells were plated before the assay at the predetermined appropriate density. After 2 days, 100 microliters of purified antibody was diluted into media, and then were transferred to the cell plates and incubated in a 8% CO₂ incubator for 5 days. The plate was then emptied by inverting and blotted dry. Room temperature DPBS containing MgCl₂ and CaCl₂ was dispensed into each well from a multichannel squeeze bottle, tapped three times, emptied by inversion and then blotted dry. 50 microliters of the fluorescent Live/Dead dye diluted in DPBS containing MgCl₂ and CaCl₂ was added to each well and incubated at 37°C. in a 5% CO₂ incubator for 30 minutes. The plates were read in a Perkin-Elmer HTS7000 fluorescence plate reader and the data was analyzed in Microsoft Excel and the results were tabulated in Table 2. The data represented an average of four experiments tested in triplicate and presented qualitatively in the following fashion: 4/4 experiments greater than threshold cytotoxicity (+++), 3/4 experiments greater than threshold cytotoxicity (++), 2/4 experiments greater than threshold cytotoxicity (+). Unmarked cells in Table 2 represented inconsistent or effects less than the threshold cytotoxicity. The 7BD-33-11A and 1A245.6 antibodies demonstrated cytotoxicity in breast and prostate tumor cell lines selectively, while having no effect on non-transformed normal cells. Both demonstrated a 25-50% greater killing than the positive control anti-Fas antibody. 11BD-2E11-2 was specifically cytotoxic in breast and ovarian cancer cells, and did not affect normal cells. The chemical cytotoxic agents induced their expected cytotoxicity while a number of other antibodies which were included for comparison also performed as expected given the limitations of biological cell assays. In total, it was shown that the three antibodies have cytotoxic activity against a number of cancer cell types. The antibodies were selective in their activity since not all cancer cell types were susceptible. Furthermore, the antibodies demonstrated functional specificity since they did not produce cytotoxicity against non-cancer cell types, which is an important factor in a therapeutic situation.

Cells were prepared for FAGS by initially washing the cell monolayer with DFBS (without Ca⁺⁺ and Mg⁺⁺). Cell dissociation buffer (INVITROGEN) was then used to dislodge the cells from their cell culture plates at 37°C. After centrifugation and collection the cells were resuspended in Dulbecco's phosphate buffered saline containing MgCl₂, CaCl₂ and 25% fetal bovine serum at 4°C (wash media) and counted, aliquoted to appropriate cell density, spun down to pellet the cells and resuspended in staining media (DPBS containing MgCl₂ and CaCl₂) containing 7BD-33-11A, 1A245.6, 11BD-2E11-2 or control antibodies (isotype control or anti-EGF-R) at 20 micrograms/mL on ice for 30 minutes. Prior to the addition of Alexa Fluor 488-conjugated secondary antibody the cells were washed once with wash media. The Alexa Fluor 488-conjugated antibody in staining media was then added for 20 minutes. The cells were then washed for the final time and resuspended in staining media containing 1 microgram/mL propidium iodide. Flow cytometric acquisition of the cells was assessed by running samples on a FACScan using the CellQuest software (BD Biosciences). The forward (FSC) and side scatter (SSC) of the cells were set by adjusting the voltage and amplitude gains on the FSC and SSC detectors. The detectors for the three fluorescence channels (FL1, FL2, and FL3) were adjusted by running cells stained with purified isotype control antibody followed by Alexa Fluor 488-conjugated secondary antibody such that cells had a uniform peak with a median fluorescent intensity of approximately 1-5 units. Live cells were acquired by gating for FSC and propidium iodide exclusion. For each sample, approximately 10,000 live cells were acquired for analysis and the resulted presented in Table 3. Table 3 tabulated the mean fluorescence intensity fold increase above isotype control and is presented qualitatively as: less than 5 (-); 5 to 50 (+); 50 to 100 (++); above 100 (+++) and in parenthesis, the percentage of cells stained.

| Table 3 | | BREAST | | | COLON | | | LUNG | OVARY | PROSTATE |
|---|---|---|---|---|---|---|---|---|---|---|
| Antibody | Isotype | MB-231 | MB-468 | MCF-7 | HT-29 | SW1116 | SW620 | NCI H460 | OVCAR | PC-3 |
| 11BD2E11-2 | IgG1, k | + (61%) | - | - | - | - | - | - | - | - |
| 7BD-33-11A | IgG2a, | k + (95%) | - | + (76%) | + (97%) | + (34%) | +(bimodal, 76%) | +(bimodal,60%) | + (51%) | + (75%) |
| 1A245.6 | IgG1, k | + (98%) | + (78%) | + (74%) | ++ | + (23%) | +(bimodal, 71%) | + (bimodal, 70%) | + (73%) | +(trimodal, 72%) |
| anti-EGFR | IgG1, k | ++ | ++bimodal | - | + (97%) | + (43%) | - | +(bimodal, 80%) | + (90%) | + (95%) |
| anti-FAS | Igm, k | - | - | - | + (30%) | - | - | + (61%) | - | - |

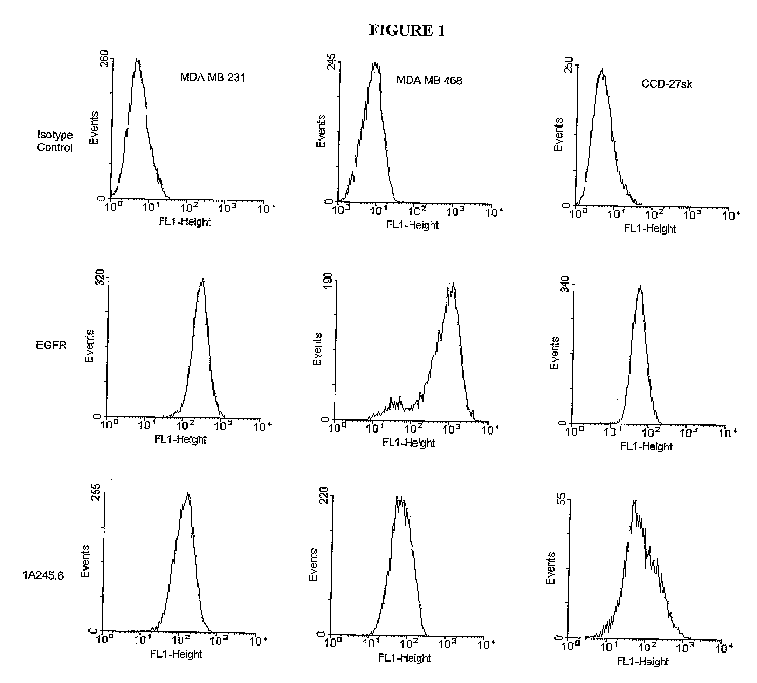

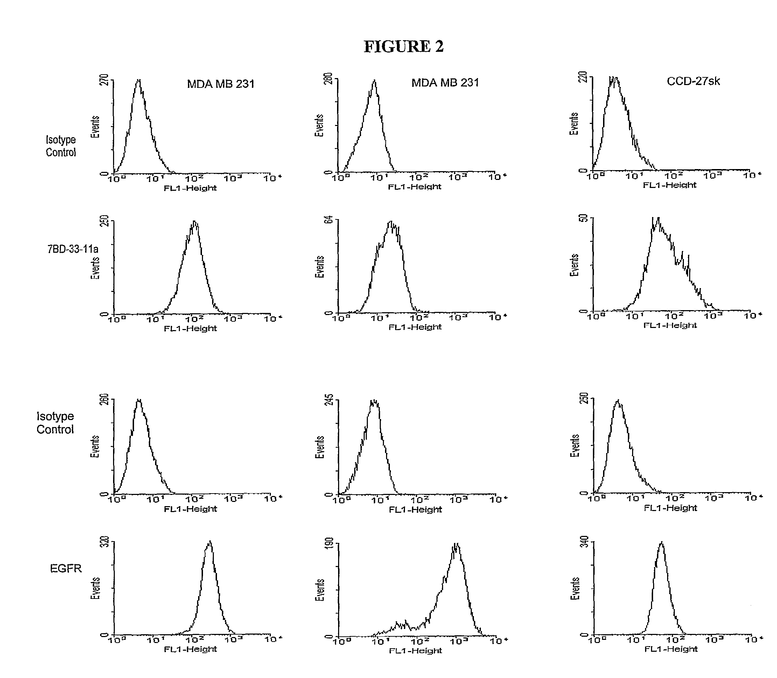

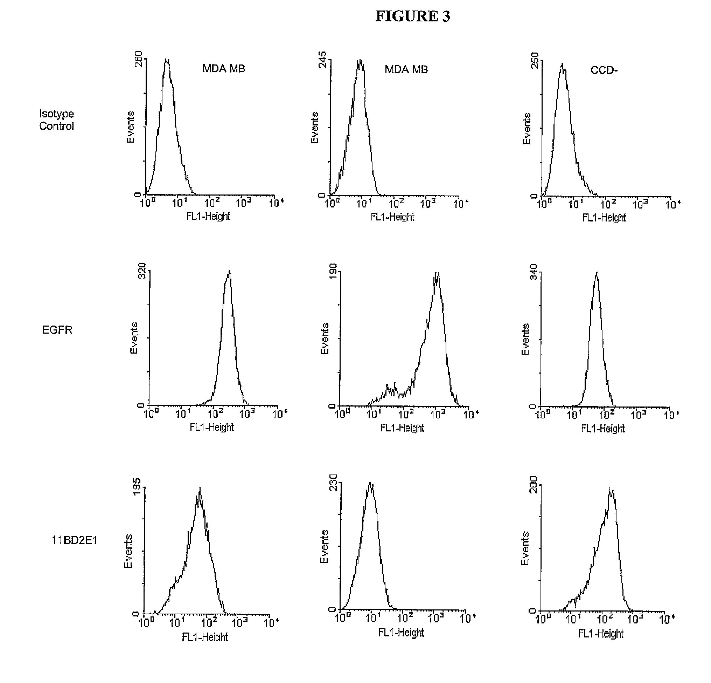

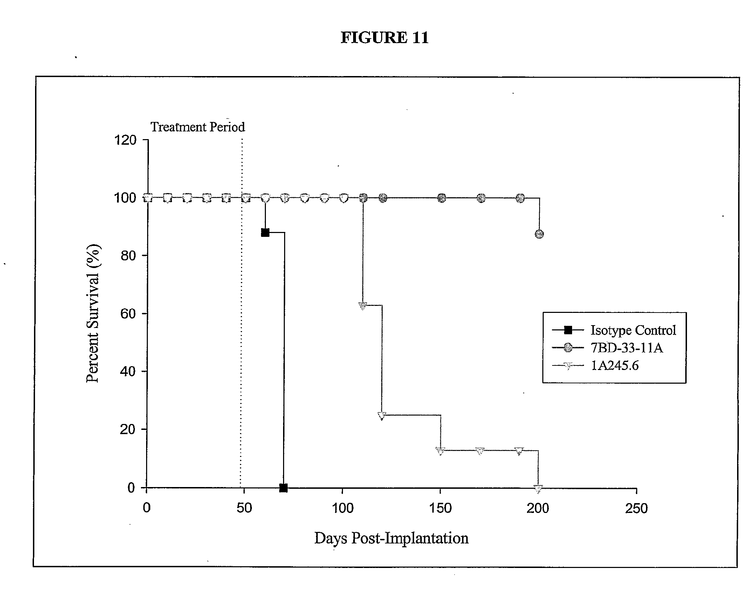

Representative histograms of 7BD-33-11A antibodies were compiled for Figure 1, 1A245.6 antibodies were compiled for Figure 2, 11BD-2E11-2 were compiled for Figure 3 and evidence the binding characteristics, inclusive of illustrated bimodal peaks, in some cases. 11BD-2E11-2 displayed specific tumor binding to the breast tumor cell line MDA-MB-231. Both 7BD-33-11A and 1A245.6 displayed similar binding to cancer lines of breast (MDA-MB-231 and MCF-7), colon, lung, ovary, and prostate origin and differential binding to one of the breast cancer cell lines (MDA-MB-468). There was binding of all three antibodies to non-cancer cells, however that binding did not produce cytotoxicity. This was further evidence that binding was not necessarily predictive of the outcome of antibody ligation of its cognate antigen, and was a non-obvious finding. This suggested that the context of antibody ligation in different cells was determinative of cytoxicity rather than just antibody binding.

### EXAMPLE 3

### In vivo experiments

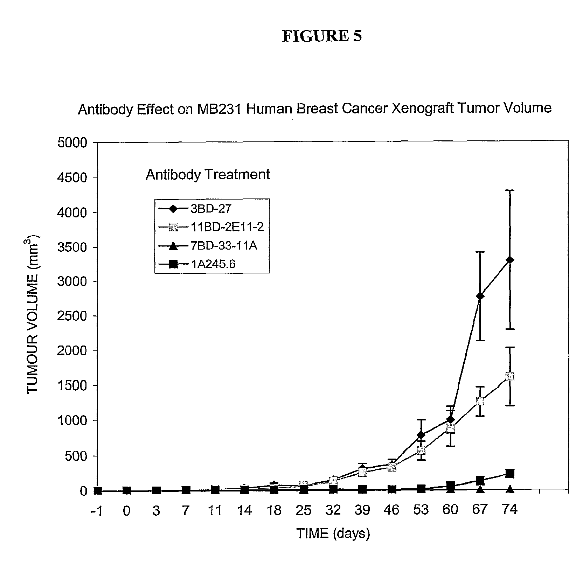

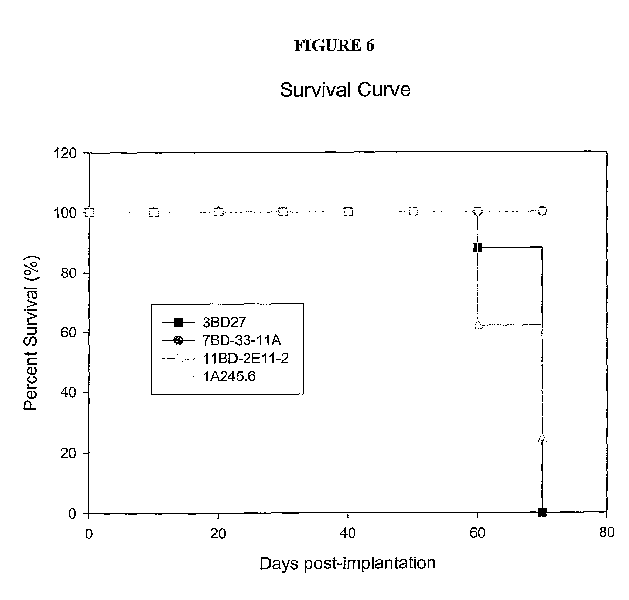

Now with reference to the data shown in Figures 5 and 6, four to eight week old, female SCID mice were implanted with 5 million MDA-MB-231 human breast cancer cells in one hundred microliters injected subcutaneously in the scruff of the neck. The mice were randomly divided into four treatment groups of ten. On the day prior to implantation 20 mg/kg of either 11BD2E-11-2, 7BD-33-11A, 1A245.6 test antibodies or 3BD-27 isotype control antibody (known not to bind MDA-MB-231 cells) were administered intrapertioneally at a volume of 300 microliters after dilution from the stock concentration with a diluent that contained 2.7 mM KCI, 1 mM KH₂PO₄, 137 mM NaCl, 20 mM Na₂HPO₄. The antibodies were then administered once per week for a period of 7 weeks in the same fashion.

Tumor growth was measured about every seventh day with calipers for up to ten weeks or until individual animals reached the Canadian Council for Animal Care (CCAC) end-points. Body weights of the animals were recorded for the duration of the study. At the end of the study all animals were euthanised according to CCAC guidelines. There were no clinical signs of toxicity throughout the study. Body weight measured at weekly intervals was a surrogate for well-being and failure to thrive. There was a minimal difference in weight for the groups treated with the isotype control, 3BD-27, and 7BD-33-11A, 1A245.6, or 11BD-2E11-2. At day 60 (11 days after the cessation of treatment) tumor volume of the group treated with 1A245.6 was 5.2% of the control group (p=0.0002) and demonstrated effectiveness at reducing tumor burden with antibody treatment. Those mice bearing cancer treated with 7BD-33-11A antibody were disease free and had no tumor burden. The tumor volume was lower in the 11BD-2E11-2 treatment group (45% of control) at day 67 (p=0.08). This also demonstrated a lesser tumor burden with cytotoxic antibody treatment in comparison to a control antibody. There was also corresponding survival benefits (Fig. 6) from treatment with 7BD-33-11A, 1A245.6, and 11BD-2E11-2 cytotoxic antibodies. The control group treated with 3BD-27 antibody reached 100% mortality by day 74 post-implantation. In contrast, groups treated with 7BD-33-11A were disease free and 1A245.6 treated animal displayed 100% survival and the group treated with 11BD-2E11-2 had 24% survival.

In total, cytotoxic antibody treatment produced a decreased tumor burden and increased survival in comparison to a control antibody in a well recognized model of human cancer disease suggesting pharmacologic and pharmaceutical benefits of these antibodies (7BD-33-11A, 1A245.6, 11BD-2E11-2) for therapy in other mammals, including man.

### EXAMPLE 4

### In vivo established tumor experiments

Five to six week old, female SCID mice were implanted with 5 million MDA-MB-231 breast cancer cells in one hundred microliters injected subcutaneously in the scruff of the neck. Tumor growth was measured with calipers every week. When the majority of the cohort reached a tumor volume of 100 mm³ (range 50-200 mm³) at 34 days post implantation 8-10 mice were randomly assigned into each of three treatment groups. 7BD-33-11A, 1A245.6 test antibodies or 3BD-27 isotype control antibody (known not to bind MDA-MB-231 cells) were administered intrapertioneally with 15 mg/kg of antibodies at a volume of 150 microliters after dilution from the stock concentration with a diluent that contained 2.7 mM KCl, 1 mM KH₂PO₄, 137 mM NaCl, 20 mM Na₂HPO₄. The antibodies were then administered three times per week for 10 doses in total in the same fashion until day 56 post-implantation. Tumor growth was measured about every seventh day with calipers until day 59 post-implantation or until individual animals reached the Canadian Council for Animal Care (CCAC) end-points. Body weights of the animals were recorded for the duration of the study. At the end of the study all animals were euthanised according to CCAC guidelines.

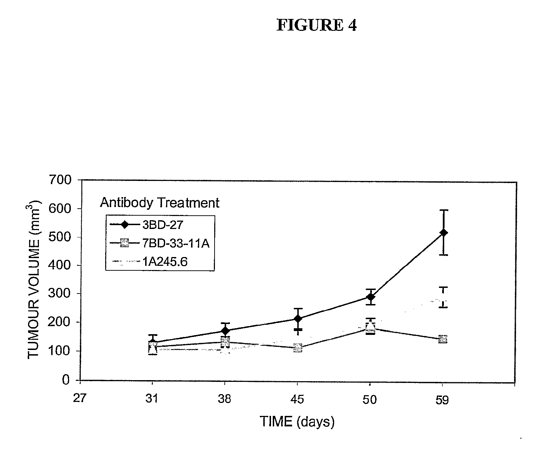

There were no clinical signs of toxicity throughout the study. Body weight was measured at weekly intervals. There was no significant difference in weight for the groups treated with the isotype control and 7BD-33-11A, or 1A245.6 antibodies. As can be seen in Figure 4, at day 59 post-implantation (2 days after the cessation of treatment), tumor volume of the group treated with 7BD-33-11A was 29.5% of the control group (p=0.0003). In this group, there was also a trend toward regression in mean tumor volume when the value for day 59 was compared to day 52 (p=0.25). Likewise, treatment with 1A245.6 antibody also significantly suppressed tumor growth and decreased tumor burdens. Animals with established tumors treated with this antibody had tumor volumes that were 56.3% of the isotype treated control group (p=0.017).

In total, treatment with 7BD-33-11A or 1A245.6 antibodies significantly decreased the tumor burden of established tumors in comparison to a control antibody in a well recognized model of human cancer disease suggesting pharmacologic and pharmaceutical benefits of these antibodies for therapy in other mammals, including man.

### EXAMPLE 5

### In Vivo MB-231 Preventative Survival Tumor Experiments

As outlined in S.N. 10/348,284, and with reference to Figure 11, 4 to 8 week old female SCID mice were implanted with 5 million MB-231 human breast cancer cells in 100 microlitres saline injected subcutaneously in the scruff of the neck. The mice were randomly divided into 3 treatment groups of 10. On the day prior to implantation, 20 mg/kg of either 7BD-33-11A, 1A245.6 test antibodies or isotype control antibody (known not to bind MB-231 or PC-3 cells) was administered intraperitoneally at a volume of 300 microliters after dilution from the stock concentration with a diluent that contained 2.7 mM KCI, 1 mM KH₂PO_{4,} 137 mM NaCl and 20 mM Na₂HPO₄. The antibodies were then administered once per week for a period of 7 weeks in the same fashion. Tumor growth was measured about every seventh day with calipers for up to 10 weeks or until individual animals reached the Canadian Council for Animal Care (CCAC) end-points. Body weights of the animals were recorded for the duration of the study. At the end of the study all animals were euthanised according to CCAC guidelines.
In continuation from S.N. 10/348,284, there was a post-treatment survival benefit (Figure 11) associated with treatment with either 7BD-33-11A or 1A245.6. 7BD-33-11A never developed tumors and only 1 mouse had died by day 200 (151 days post-treatment). In contrast, all of the isotype control mice had died by day 23 post-treatment. The 1A245.6 treated group did not reach 100 percent mortality until day 151 post-treatment which is greater than 6 times longer than the isotype control treatment group. In summary 1A245.6 and 7BD-33-11A increased survival and decreased tumor burden in a breast tumor model of human cancer.

### EXAMPLE 6

### In Vivo MB-231 Established Tumor Experiments

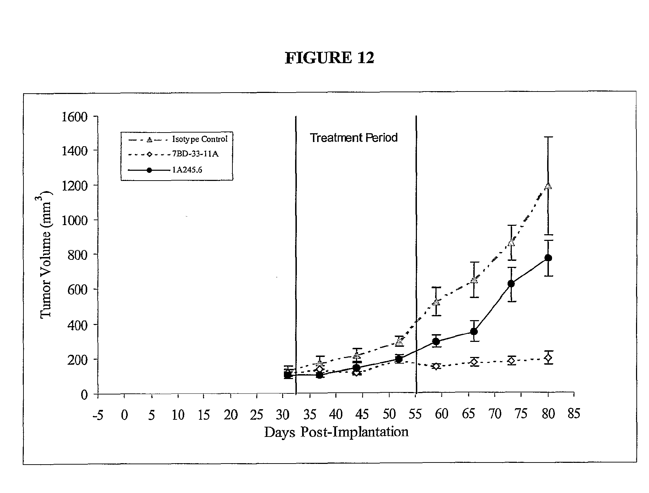

As outlined in S.N. 10/348,284, and with reference to Figures 12 and 13, 5 to 6 week old female SCID mice were implanted with 5 million MB-231 human breast cancer cells in 100 microlitres saline injected subcutaneously in the scruff of the neck. Tumor growth was measured with calipers every week. When the majority of the cohort reached a tumor volume of 100 mm³ (range 50-200 mm³) at 34 days post-implantation 8-10 mice were randomly assigned into each of 3 treatment groups. 7BD-33-11A, 1A245.6 test antibodies or isotype control antibody was administered intraperitoneally with 15 mg/kg of antibodies at a volume of 150 microliters after dilution from the stock concentration with a diluent that contained 2.7 mM KCI, 1 mM KH₂PO₄, 137 mM NaCl and 20 mM Na₂HPO₄. The antibodies were then administered 3 times per week for 10 doses in total in the same fashion until day 56 post-implantation. Tumor growth was measured about every seventh day with calipers until day 59 post-implantation or until individual animals reached the CCAC end-points. Body weights of the animals were recorded for the duration of the study. At the end of the study all animals were euthanised according to CCAC guidelines.

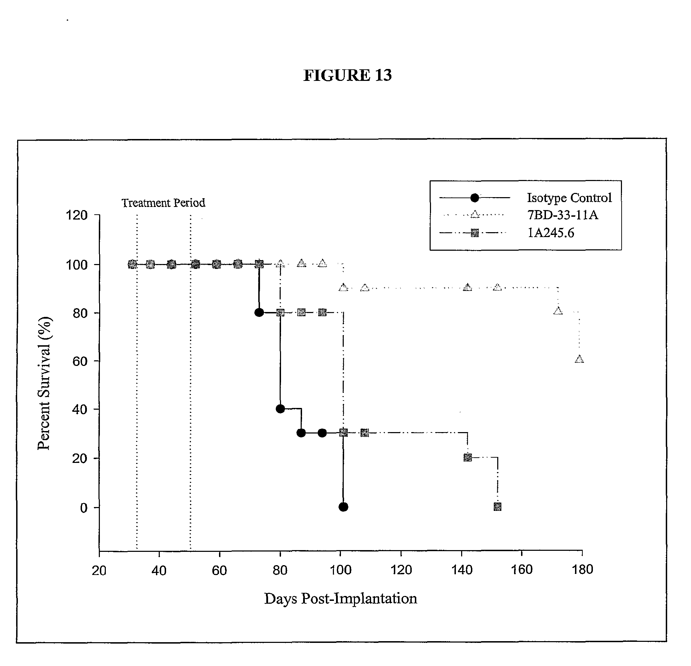

In continuation from S.N. 10/348,284, there was a post-treatment tumor burden reduction (Figure 12) and survival benefit (Figure 13) associated with treatment with either 7BD-33-11A or 1A245.6. At day 80 (23 days post-treatment) both 7BD-33-11A and 1A245.6 had decreased mean tumor volumes compared to isotype control treatment; 83 (p=.001) and 35 percent (p=.135) respectively. A *Coxproportional model* was used to compare the hazard (risk) rates in the different groups. In this method, the hazard rate of every group is compared with the hazard of the isotype control group. At approximately 60 days post-treatment, the risk of dying in the 7BD-33-11A group was 16 percent in comparison to the isotype control treatment group (p=.0006). The survival benefit associated with 7BD-33-11A appeared to continue on well past the 100 day post-treatment mark. At day 130 post-treatment, 7BD-33-11A had 60% survival while all of the isotype control mice had died at day 50 post-treatment. 1A245.6 had double the survival time in comparison to the isotype control: 100 versus 50 days post-treatment. Therefore both 7BD-33-11A and 1A245.6 lowered the tumor burden and increased survival in comparison to a control antibody in a well recognized model of human breast cancer disease suggesting pharmacologic and pharmaceutical benefits of these antibodies for therapy in other mammals, including man.

### EXAMPLE 7

### In Vivo PC-3 Preventative Tumor Experiments

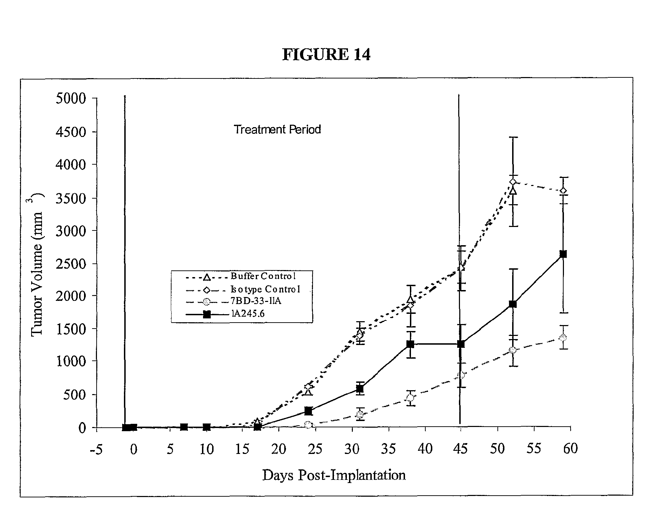

With reference to the data shown in Figures 14 and 15, 4 to 8 week old, male SCID mice were implanted with 1 million PC-3 human prostate cancer cells in 100 microliters saline injected subcutaneously in the scruff of the neck. The mice were randomly divided into 4 treatment groups of 8. On the day prior to implantation 20 mg/kg of 7BD-33-11A or 1A245.6 test antibody or isotype control antibody or buffer control was administered intraperitoneally at a volume of 300 microliters after dilution from the stock concentration with a diluent that contained 2.7 mM KCI, 1 mM KH₂PO₄, 137 mM NaCl and 20 mM Na₂HPO₄ The antibodies and buffer control were then administered once per week for a period of 7 weeks in the same fashion. Tumor growth was measured about every 7th day with calipers for up to 10 weeks or until individual animals reached the CCAC end-points or day 52. Body weights of the animals were recorded for the duration of the study. At the end of the study all animals were euthanised according to CCAC guidelines.

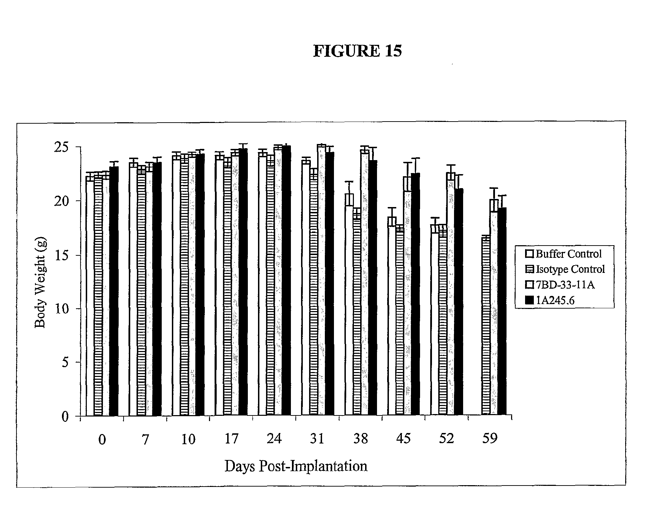

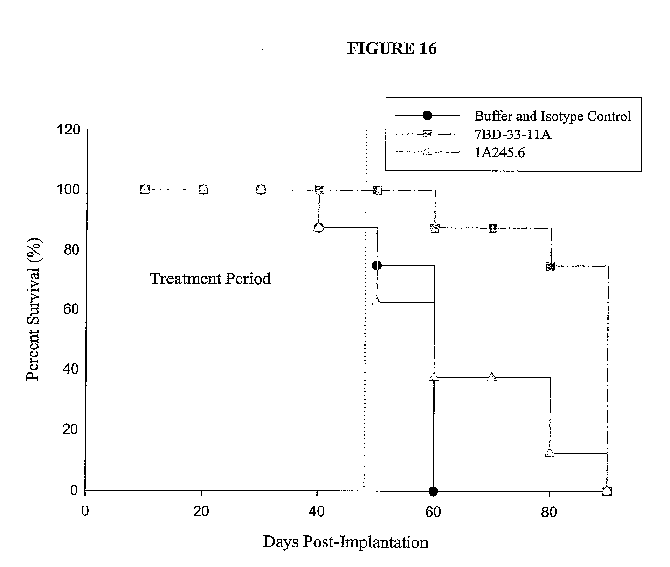

Using the least significant difference method (LSD) to specify the different groups, it is apparent that both 7BD-33-11A and 1A245.6 significantly reduced the tumor burden in treated mice in comparison to controls (Figure 14). After treatment (day 52), 7BD-33-11A prevented tumor growth by 69 percent (p=.001) in comparison to isotype control and 1A245.6 also prevented tumor growth by 50 percent (p=.017) in comparison to isotype control. Similar findings were found when comparisons were made to the buffer control. In a PC-3 prostate cancer xenograft model, body weight can be used as a surrogate indicator of disease progression (Figure 15). A repeated analysis of variance (Rep. ANOVA) indicates there was no significant difference in body weight between the isotype and buffer control group. Analysis of variance determined that at day 52, 7BD-33-11A had a significantly higher body weight than both of the control groups and the 1A245.6 treated group (p<.03). Overall, 7BD-33-11A prevented body weight loss by 54 percent (p=.002) while 1A245.6 prevented body weight loss by 25 percent (p=.004) compared to the isotype control group. Mice were monitored post-treatment for survival (Figure 16). 100 percent of 7BD-33-11A and 1A245.6 treated mice reached mortality by day 38 post-treatment, which is greater than 3 times longer than the isotype and buffer control treatment group, 11 days post-treatment.

In summary, 7BD-33-11A and 1A245.6 antibody treatment reduced tumor burden, delayed disease progression and extended survival in comparison to an isotype control antibody and a buffer control in a well-recognized model of human prostate cancer. These results suggest a potential pharmacologic and pharmaceutical benefit of these antibodies (7BD-33-11A and 1A245.6) as a therapy beyond breast cancer.

### EXAMPLE 8

### In Vivo PC-3 Established Tumor Experiments:

Male SCID mice, 4 to 8 weeks old, were implanted with 1 million PC-3 prostate cancer cells in 100 microliters saline injected subcutaneously in the scruff of the neck. Tumor growth was measured with calipers every week. When the majority of the cohort reached a tumor volume of 275 mm³ (range 144-406 mm³) at 21 days post implantation, 9-10 mice were randomized into each of 4 treatment groups. 7BD-33-11A or 1A245.6 or isotype control antibody was administered intraperitoneally with 20 mg/kg/dose at a volume of 300 microliters after dilution from the stock concentration with a diluent that contained 2.7 mM KCI, 1 mM KH₂PO₄, 137 mM NaCl and 20 mM Na₂HPO₄. The antibodies were then administered 3 times per week for a total of 10 doses in the same fashion until day 43 post-implantation. Tumor growth was measured about every seventh day with calipers for the duration of the study or until individual animals reached CCAC end-points. Body weights of the animals were recorded for the duration of the study. At the end of the study all animals were euthanised according to CCAC guidelines.

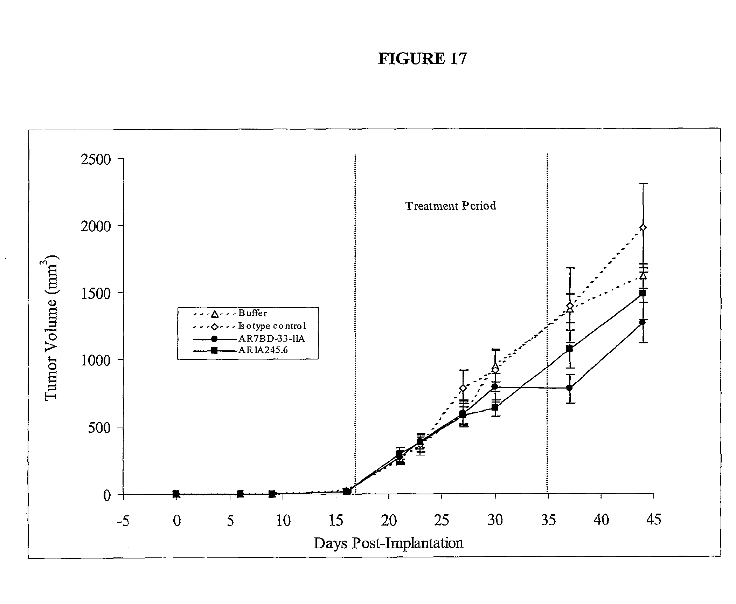

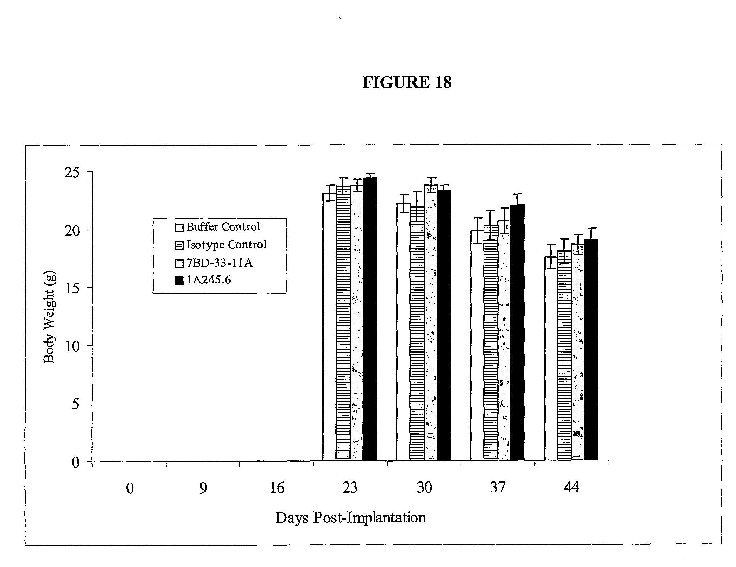

At the time of randomization the mean tumor volumes and the standard deviations in each group were similar. Statistically there was no difference in body weight between the groups. This indicated that true randomization had occurred. As shown in Figure 17, the antibody 7BD-33-11A was able to significantly suppress tumor growth by 36 percent (p=.024) in comparison to isotype control at the end of the 3-week treatment period. 1A245.6 showed no significant difference when compared to isotype or buffer control treatment groups. Likewise, neither 7BD-33-11A or 1A245.6 showed any significant difference in comparison to isotype or buffer control treatment groups in terms of body weight (Figure 18). All groups displayed the same significant amount of body weight loss throughout the study (p<.001).

In summary, 7BD-33-11A is significantly more effective than the isotype control antibody in suppressing tumor growth in an established tumor xenograft model of prostate cancer in SCID mice. Therefore treatment with 7BD-33-11A significantly decreased the tumor burden of established tumors in two well-recognized models of human cancer disease (breast and prostate) suggesting pharmacologic and pharmaceutical benefits of this antibody for therapy in other mammals, including man.

### EXAMPLE 9

### Normal Human Tissue Staining

IHC studies were conducted to characterize the 7BD-33-11A and 1A245.6 antigen distribution in humans. IHC optimization studies were performed previously in order to determine the conditions for further experiments. 7BD-33-11A and 1A245.6 monoclonal antibody was produced and purified as stated above.

Tissue sections were deparaffinized by drying in an oven at 58°C for 1 hour and dewaxed by immersing in xylene 5 times for 4 minutes each in Coplin jars. Following treatment through a series of graded ethanol washes (100%-75%) the sections were rehydrated in water. The slides were immersed in 10 mM citrate buffer at pH 6 (Dako, Toronto, Ontario) then microwaved at high, medium, and low power settings for 5 minutes each and finally immersed in cold PBS. Slides were then immersed in 3% hydrogen peroxide solution for 6 minutes, washed with PBS three times for 5 minutes each, dried, incubated with Universal blocking solution (Dako, Toronto, Ontario) for 5 minutes at room temperature. 7BD-33-11A, 1A245.6, monoclonal mouse anti-vimentin (Dako, Toronto, Ontario) or isotype control antibody (directed towards *Aspergillus niger* glucose oxidase, an enzyme which is neither present nor inducible in mammalian tissues; Dako, Toronto, Ontario) were diluted in antibody dilution buffer (Dako, Toronto, Ontario) to its working concentration (5µg/mL for each antibody) and incubated overnight for 1 hour at room temperature. The slides were washed with PBS 3 times for 5 minutes each. Immunoreactivity of the primary antibodies was detected/visualized with HRP conjugated secondary antibodies as supplied (Dako Envision System, Toronto, Ontario) for 30 minutes at room temperature. Following this step the slides were washed with PBS 3 times for 5 minutes each and a color reaction developed by adding DAB (3,3'-diaminobenzidine tetrahydrachloride, Dako, Toronto, Ontario) chromogen substrate solution for immunoperoxidase staining for 10 minutes at room temperature. Washing the slides in tap water terminated the chromogenic reaction. Following counterstaining with Meyer's Hematoxylin (Sigma Diagnostics, Oakville, ON), the slides were dehyrdated with graded ethanols (75-100%) and cleared with xylene. Using mounting media (Dako Faramount, Toronto, Ontario) the slides were coverslipped. Slides were microscopically examined using an Axiovert 200 (Zeiss Canada, Toronto, ON) and digital images acquired and stored using Northern Eclipse Imaging Software (Mississauga, ON). Results were read, scored and interpreted by a pathologist.

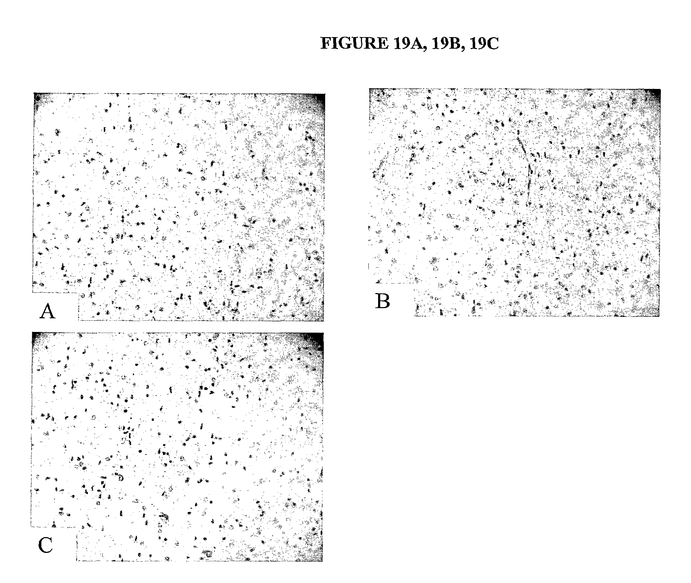

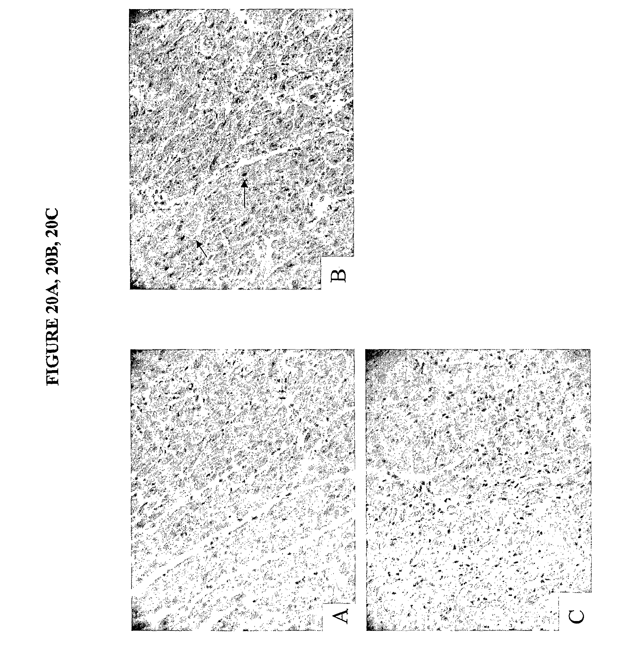

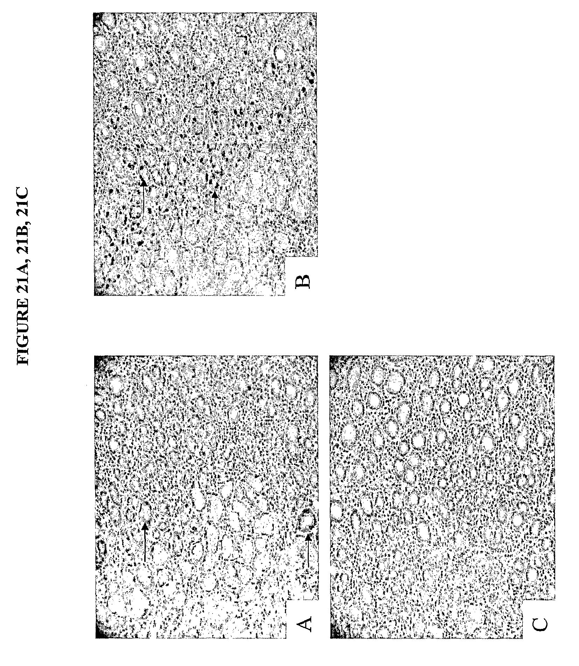

Binding of antibodies to 59 normal human tissues was performed using a human, normal organ tissue array (Imgenex, San Diego, CA). Table 4 presents a summary of the results of 7BD-33-11A and 1A245.6 staining of an array of normal human tissues. From the table, there are 3 categories of tissue staining. A group of tissues was completely negative. These tissues included normal skin, brain (Figure 19A), ovary, thymus, thyroid, small bowel, esophaguas, heart (Figure 20A), gall bladder and lymph node for 7BD-33-11A. For 1A245.6, the completely negative tissues comprised of skin, sub-cutis fat, esophagus and brain (Figure 19B). A second group of tissues comprised tissues that demonstrated positive staining. These included the liver and pancreas for 7BD-33-11A. The tonsil had the strongest staining with this antibody. For 1A245.6, positive staining occurred in the liver, heart (Figure 20B), testis, thyroid, adrenal gland and myometrium. As with 7BD-33-11A, 1A245.6 stained the tonsil the strongest. A third group of tissues included tissues in which staining was positive in the tissue section, but was limited to infiltrating macrophages, lymphocytes, fibroblasts or the epithelium, for example the stomach for both 7BD-33-11A and 1A245.6 (Figure21A and B respectively). It should be noted that the 7BD-33-11A antigen is not present on cells of several of the vital organs, including kidney, heart (Figure 20A) and lung. Overall, 7BD-33-11A binds to a smaller subset of normal human tissues compared to 1A245.6 with weak to moderate binding in the tissues that are positive. 1A245.6 staining, albeit more extensive, is also generally weak to moderate in intensity and in the majority of cases is limited to the epithelium of the stained tissue. These results suggest that the antigen for 7BD-33-11A is not widely expressed on normal tissues, and that the antibody would bind specifically to a limited number of tissues in humans. In addition, the antigen for 1A245.6, besides being present in the heart and liver, is limited to epithelium and infiltrating lymphocytes, macrophages and fibroblasts.

**Table 4: IHC On Normal Human Tissue**

| Sec No. | Organ | 1A245.6 | 7BD-33-11A | Vimentin |
|---|---|---|---|---|
| 1 | *Skin | - | - | +++ Fibroblasts |
| 2 | *Skin | - | - | +++ Fibroblasts |
| 3 | Sub-cutis fat | - | - | ++ Adipocytes |
| 4 | Breast | +/- Fibroblasts +/- Fibroblasts | - | ++ Endothelium, Smooth muscles of blood vessels |
| 5 | Breast | +++Lobular epithelium | +/- Filrcblasts +/- Filrcblasts | ++ Blood vessles, stroma |
| 6 | Spleen | ++Lymphocytes | +/- Lymphocytes | +++ Sinusoidal endothelium, Lymphocytes |
| 7 | Spleen | +++Lymphocytes | +/- Lymphocytes | +++ Sinusoidal endothelium, Lymphocytes |
| 8 | Lymphnode | ++Endothelium of blood vessels, Lymphocytes | - | +++ Lymphocytes |
| 9 | Lymphnode | + Bndothelium of blood vessels | - | ++ Blood vessls, Lymphocytes |
| 10 | Skeletal muscle | +/- Endothelium of blood vessels | - | +/- Blood vessels |
| 11 | Nasal mucosa | -NR | - NR | - NR |
| 12 | Lung | +/- Interstitial cells (macrophages) | **- | ++ Alevolar epithelium, Macrophages |
| 13 | Lung | +/-Bronchiolar epithelium,++Macrophages | + Macrophages | ++Alevolar epithelium, Macrophages, Lymphocytes |
| 14 | Bronchus | - NR | - NR | ++ Chondrocytes, NR |
| 15 | Heart | ++ Cardiac muscle | ****-** | +++ Blood vessles |
| 16 | S₃livary gland | ++ Acinar epithelium | + Acinar epithelium | +++ Blood vessels, Peripheralnerves |
| 17 | Liver | +++ Hepatocytes | ++ Hepatocytes | ++Blood vessels |
| 18 | Liver | +++ Hepatocytes | + Hepatocytes | =+++ Blood vessles, Macrophages |
| 19 | Liver | + Hepatocytes | +/- Hepatocytes | +/- Blood vessels |
| 20 | Gall Hadder | ++ Mucosal epithelium, Fibroblasts, Smooth muscle fibers | - | +++ Lymphocytes |
| 21 | Pancreas | +++ Acinar epithelium, Islets of Langherhans | + Acinar epithelium | +++ Acinar epithelium, Blood vessels |
| 22 | Pancreas | +++ Acinar epithelium, Islets of Langherhans | ++ Acinarepithelium, Islets of Langherhans | ++ Acinar epithelium, Blood vessels |
| 23 | Tonsil | +++ Keratin, +Lymphocytes | +++ Keratin, +/- Lymphocytes | +++ Lymphocytes |
| 24 | Esophagus | - | - | ++ Blood vessels |
| 25 | Esophagus | - | - | +++ Blood vessels |
| 26 | ***Stomach body | +/- Gastric gland epithelium, ++ Lymphocytes in laminapropria | ++ Gastric gland epithelium | +++ Blood vessels & fibroblasts |
| 27 | ***Stomach body | +++ Gastric gland epithelium, +Lymphocytes in laminapropria | ++ Gastric gland epithelium | +++ Lymphocytes, Blood vessels, Fibroblasts |
| 28 | Stomach antrum | +/- Gastric glandepithelium,+Lymphocytes | ++ Gastric gland epithelium | +++ Lymphocytes |
| 29 | Stomach, Smooth muscle | - | - | +++ Lymphocytes, Blood vessels |
| 30 | Duodenum | +++ Lymphocytes | + Intestinal gland epithelium | ++ Fibroblasts, Blood vessels |
| 31 | Small bowel | +/-Lymphocytes in lamina propria | - | + Lymphocytes, Blood vessels |
| 32 | Small bowel | +/-Lymphocytes in lamina propria | - | +++ Lymphocytes in lamina propria |
| 33 | Appendix | +++ Mucosal epithelium, Lymphocytes | ++ Lymphocytes | +++ Lymphocytes, Blood vessels |
| 34 | Colon | +/- Lymphocytes | +/- Macrophages in lamina propria | ++ Lymphocytes |
| 35 | Col on | + Lymphocytes | - | ++ Lymphocytes, Blood vessels |
| 36 | Rectum | +/- Lymphocytes, Bloodvessels | - | ++ Lymphocytes, Blood vessels |
| 37 | Kidney cortex | ++Tubular epithelium | - | +++ Glomerular Cspillary, Blood vessels |
| 38 | Kidney cortex | +++ Tubular epithelium | + Tubularepithelium | +++ Tubular epitheliumglomerular capillary, Blood vessels |
| 39 | Kidney medulla | ++ Tubular epithelium | - | ++ Renal tubule epithelium, Lipocytes, fibroblasts |
| 40 | Urinary Hadder | ++ Transitional epithelium | +/- Transitional epithelium | ++ Blood vessels |
| 41 | Prostate | +++ Glandular epithelium | ++ Gandular epithelium | +++ Glandular epithelium, Blood vessels |
| 42 | Prostate | +++ Glandular epithelium | ++ Gandular epithelium | +++ Glandular epithelium, Blood vessels |
| 43 | Seminalvesicle | + Mucosal epithelium | +/- Mucosal epithelium | +/- |
| 44 | Testis | ++ Germinal epithelium, + Leydig cells | +/- Leydig cells | +++ Germinal epithelium |
| 45 | Endometrium profilarative | +++ Glandular epithelium, + Stroma | - | +++ Endometrial glands, Stroma |
| 46 | Endometrium secretory | ++ Glandular epithelium, Stroma | - | +- Glandular epithelium, +++ Blood vessels |
| 47 | myometrium | + Smooth muscle fibers | +/- Fibroblasts | ++ Smooth muscle fibers, Blood vessels |
| 48 | Uterine cervix | +/- Fibroblasts | - | +++Fibroblasts |
| 49 | Salpinx | + Mucosal epithelium, Blood vessels | - | +/- Mucosal epithelium, +++ Blood vessels |
| 50 | ****Ovary | + Stromal cells | - | +++ Stromalcells |
| 51 | Placenta villi | + Trophoblasts | - | ++ Blood vessels |
| 52 | Placenta, villi | ++ Trophoblasts | - | ++ Blood vessels |
| 53 | Umbilical cord | - | - | ++ Fibroblasts |
| 54 | Adrenal gland | ++ Endocrine cells | **+/- | **+/- |
| 55 | Thyroid | +/- Follicular cells | - | ++ Follicular cells, Blood vessels |
| 56 | Thymus | + Lymphocytes | - | +++ Lymphocytes |
| 57 | Brain white matter | - | - | ++ Astrocytes |
| 58 | Brain gray matter | - | - | ++ Blood vessels |
| 59 | Cerebellum | - | - | ++ Cerebellar cortex |

| | | | | |
|---|---|---|---|---|
| Abbreviations: * : Originally pigmented stratum basale; ** : Endogenous cytoplasmic pigment/back ground staining; *** : Stomach antrum (not stomach body); **** : Ovarian stroma only; NR : The section is not representative; CS : The section is completely sloughed. | | | | |

### EXAMPLE 10

### Human Tumor Tissue Staining

An IHC study was undertaken to determine the cancer association of the 7BD-33-11A and 1A245.6 antigen with human breast cancers and whether either antibody was likely to recognize human cancers. A comparison was made for vimentin (positive control), and an antibody directed towards *Aspergillus niger* glucose oxidase, an enzyme which is neither present nor inducible in mammalian tissues (negative control). A breast cancer tissue array derived from 50 breast cancer patients and 10 samples derived from non-neoplastic breast tissue in breast cancer patients were used (Imgenex Corporation, San Diego, CA). The following information was provided for each patient: age, sex, and diagnosis. The procedure for IHC from Example 9 was followed. All antibodies were used at a working concentration of 5 µg/ml.

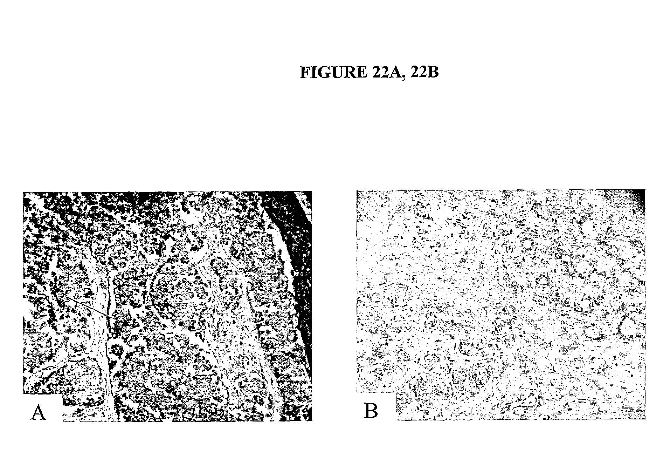

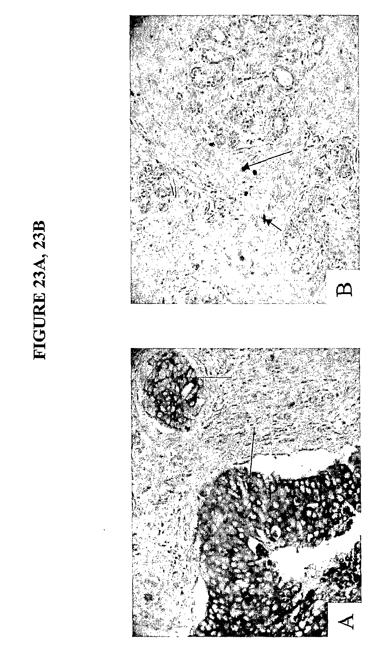

Table 5 provides a binding summary of 7BD-33-11A and 1A245.6 antibody staining of a breast cancer tissue array. Each array contained tumor samples from 50 individual patients. Overall, 36 percent of the 50 patients tested were positive for the 7BD-33-11A antigen (Figure 22A) compared to 98 percent for 1A245.6 (Figure 23A). For 7BD-33-11A, 0 out of 10 normal breast tissue samples from breast cancer patients were positive (Figure 22B). Conversely, 9 out of 10 normal breast tissue samples were positive for 1A245.6. However, staining was due to infiltrating fibroblasts in the majority of cases (Figure 23B). No correlation between estrogen and progesterone receptor status was evident for 1A245.6 (Table 6). There were a slightly higher number of positive 7BD-33-11A antigen tissues that were also estrogen and progesterone receptor expressers (Table 7). For the 7BD-33-11A antigen, it also appeared there was a trend to greater positive expression with higher tumor stage (Table 7) and for 1A245.6, the intensity of tissue staining appeared to correlate with higher tumor stage (Table 6). Both the 7BD-33-11A and 1A2425.6 staining was specific for cancerous cells and staining occurred on both the membrane and within the cytoplasm. The staining pattern, from both 7BD-33-11A and 1A245.6, showed that in patient samples, the antibody is highly specific for malignant cells and the respective antigens are present on the cell membrane thereby making it an attractive druggable target.

**Table 5: IHC On Human Breast Tumor Tissue**

| Sec. No. | Age | Sex | Diagnosis | 1A245.6 | 7BD-33-11A |
|---|---|---|---|---|---|
| 1 | F | 28 | Infiltrating duct carcinoma | +++ MC | ++ MC |
| 2 | F | 71 | Solid papillary carcinoma | +++ MC | +/- |
| 3 | F | 26 | Infiltrating duct carcinoma | ++ MC | - |
| 4 | F | 43 | Infiltrating duct carcinoma | ++ MC | +/- |
| 5 | F | 39 | infiltrating duct carcinoma | + MC Tumor, +++ Necrotic area | +/- |
| 6 | F | 46 | Ductal carcinoma *in situ* | ++ MC | +/- |
| 7 | F | 47 | Infiltrating duct carcinoma | +++ MC Tumor. ++ stroma | + MC |
| 8 | M | 67 | Infiltrating duct carcinoma | +++ MC | + MC |
| 9 | F | 33 | Infiltrating duct carcinoma | ++ MC | - |
| 10 | F | 47 | Infiltrating duct carcinoma | ++ MC | - |
| 11 | F | 49 | Invasive lobular carcinoma | -Tumor, +/- Fibroblasts | - |
| 12 | F | 46 | Infiltrating duct carcinoma | +++ MC | - |
| 13 | F | 39 | Infiltrating duct carcinoma | ++ MC | - |
| 14 | F | 43 | Infiltrating Iobularcarcinoma | +++ MC | +/- |
| 15 | F | 54 | Infiltrating lobular carcinoma | ++ MC | +/- |
| 16 | F | 58 | Infiltrating duct carcinoma | ++ MC Tumor, Stroma, +++ Necrotic area | +/- |
| 17 | F | 37 | Infiltraling duct carcinoma | +++ MC | - |
| 18 | F | 43 | Infiltrating duct carcinoma | +++ MC Tumor, Stroma | +++ M/C |
| 19 | F | 51 | Infiltrating duct carcinoma | +++ MC | + MC |
| 20 | F | 80 | Medullary carcinoma | ++ MC | - |
| 21 | F | 36 | Infiltrating duct carcinoma | ++ MC Tumor, Stroma | - |
| 22 | F | 59 | Infiltrating duct carcinoma | + MC | +/- Blood vessels |
| 23 | F | 34 | Ductal carcinoma *in situ* | ++ MC Tumor, Sroma, +++ Necrotic area | + Tumor, ++ Necrotic area |
| 24 | F | 54 | Infiltrating duct carcinoma | ++ MC | +/- |
| 25 | F | 47 | Infiltrating duct carcinoma | +++ MC | ++ MC |
| 26 | F | 53 | infiltrating duct carcinoma | ++ MC | - |
| 27 | F | 59 | Infiltrating duct carcinoma | + MCTumor, Stroma, Endothelium of blood vessels | +/- Necrotic area |
| 28 | F | 60 | Signet ring cell carcinoma | +++ MC | - |
| 29 | F | 37 | infiltrating duct carcinoma | +++ MC | ++ MC |
| 30 | F | 46 | Infiltrating duct carcinoma | ++ MC | +/- |
| 31 | F | 35 | Infiltrating duct carcinoma | +/- | - |
| 32 | F | 47 | Infiltrating duct carcinoma | +Tumor, +++ Necrotic area | - |
| 33 | F | 54 | Infiltrating duct carcinoma | ++ MC | - |
| 34 | F | 47 | Infiltrating duct carcinoma | + MC Tumor, Stroma | - |
| 35 | F | 41 | Infiltrating duct carcinoma | +++ MC | - |
| 36 | F | 38 | Infiltrating duct carcinoma | +++ MC | - |
| 37 | F | 55 | Infiltrating duct carcinoma | + MC Tumor, Stroma | - |
| 38 | F | 65 | Infiltrating duct carcinoma | ++ MC Tumor, Stroma | - |
| 39 | M | 66 | Infiltrating duct carcinoma | + MC Tumor, Stroma | - |
| 40 | F | 44 | Infiltrating duct carcinoma | ++ MC | - |
| 41 | F | 52 | Metastatic carcinoma in lymph node | ++ MC | - |
| 42 | F | 32 | Metastatic carcinoma in lymph node | ++ MC | - |
| 43 | F | 58 | Metastatic carcinoma in lymph node | +++ MC | +/- |
| 44 | F | 52 | Metastatic carcinoma in lymph node | ++ MC | - |
| 45 | F | 58 | Metastatic carcinoma in lymph node | + MC | - |
| 46 | F | 38 | Metastatic carcinoma in lymph node | +++ MC | - |
| 47 | F | 45 | Metastatic carcinoma in lymph node | +/- | - |
| 48 | F | 45 | Metastatic carcinoma in lymph node | ++ MC | - |
| 49 | F | 29 | Metastatic carcinoma in lymph node | ++ MC | - |
| 50 | F | 61 | Metastatic carcinoma in lymph node | ++ MC | - |
| *51 | F | 46 | Nipple | ++ Sebaceous glands | - |
| *52 | F | 47 | Nipple | ++ MC | - |
| *53 | F | 40 | Normal Breast | - | - |
| *54 | F | 43 | Normal Breast | +/- Fibroblasts | - |
| *55 | F | 40 | Normal Breast | ++ Lobular epithelium, Fibroblasts, Endothelium | - |
| *56 | F | 40 | Normal Breast | +/- Fibroblasts | - |
| *57 | F | 45 | Normal Breast | +/. Fibroblasts | - |
| *58 | F | 44 | Normal Breast | +/- Fibroblasts | - |
| *59 | F | 37 | Normal Breast | ++ Lobular epithelium, Fibroblasts | - |
| *60 | F | 51 | Normal Breast | +/- Fibroblasts | - |

| | | | | | |
|---|---|---|---|---|---|
| Abbreviations: PS : the section is partially detached; * : Non-neoplastic breast tissue in breast cancer patient. | | | | | |

**Table 6: IHC Correlation Summary For 1A245.6**

| | | | Binding Score | | | | | | |
|---|---|---|---|---|---|---|---|---|---|
| | | Total # | - | +/- | + | ++ | +++ | Total positive | % positive of total |
| PatientSamples | Tumor | 50 | 1 | 2 | 8 | 23 | 16 | 49 | 98% |
| | Normal | 10 | 1 | 5(Fibroblasts) | 0 | 4 | 0 | 9 | 90% |
| ER Status | ER+ | 28 | 0 | 1 | 2 | 14 | 11 | 28 | 100% |
| | ER- | 22 | 1 | 1 | 6 | 9 | 5 | 21 | 96% |
| | Unknown | 0 | 0 | 0 | 0 | 0 | 0 | 0 | 0% |
| PR Status | PR+ | 19 | 0 | 0 | 1 | 8 | 10 | 19 | 100% |
| | PR- | 30 | 1 | 2 | 7 | 14 | 6 | 29 | 97% |
| | Unknown | 1 | 0 | 0 | 0 | 0 | 0 | 1 | 100% |
| AJCCTumorStage | T1 | 4 | 0 | 1 | 1 | 1 | 1 | 4 | 100% |
| | T2 | 21 | 1 | 0 | 6 | 9 | 5 | 20 | 95% |
| | T3 | 20 | 0 | 1 | 1 | 10 | 8 | 20 | 100% |
| | T4 | 5 | 0 | 0 | 0 | 3 | 2 | 5 | 100% |

**Table 7: IHC Correlation Summary For 7BD-33-11A**

| | | | Binding Score | | | | | | |
|---|---|---|---|---|---|---|---|---|---|
| | | Total # | - | +/- | + | ++ | +++ | Total positive | % positive of total |
| PatientSamples | Tumor | 50 | 30 | 12 | 4 | 3 | 1 | 20 | 40% |
| | Normal | 10 | 10 | 0 | 0 | 0 | 0 | 0 | 0% |
| ER Status | ER+ | 28 | 16 | 9 | 1 | 2 | 0 | 12 | 43% |
| | ER- | 22 | 15 | 3 | 2 | 1 | 1 | 7 | 32% |
| | Unknown | 0 | 0 | 0 | 0 | 0 | 0 | 0 | 0% |
| PR Status | PR+ | 19 | 9 | 6 | 2 | 2 | 0 | 10 | 53% |
| | PR- | 30 | 20 | 6 | 2 | 1 | 1 | 10 | 33% |
| | Unknown | 1 | 1 | 0 | 0 | 0 | 0 | 0 | 0% |
| AJCC Tumor Stage | T1 | 4 | 4 | 0 | 0 | 0 | 0 | 0 | 0% |
| | T2 | 21 | 14 | 3 | 2 | 1 | 1 | 7 | 33% |
| | T3 | 20 | 11 | 6 | 2 | 1 | 0 | 9 | 45% |
| | T4 | 5 | 1 | 3 | 0 | 1 | 0 | 4 | 80% |

To determine whether either the 7BD-33-11A or 1A245.6 antigen is expressed on other human cancer tissues in addition to breast cancer, both antibodies were individually tested on a multiple human tumor tissue array (Imgenex, San Diego, CA). The following information was provided for each patient: age, sex, organ and diagnosis. The staining procedure used was the same as the one outlined in Example 9. Vimentin was used as a positive control antibody and the same negative control antibody was used as described for the human breast tumor tissue array. All antibodies were used at a working concentration of 5 µg/mL.

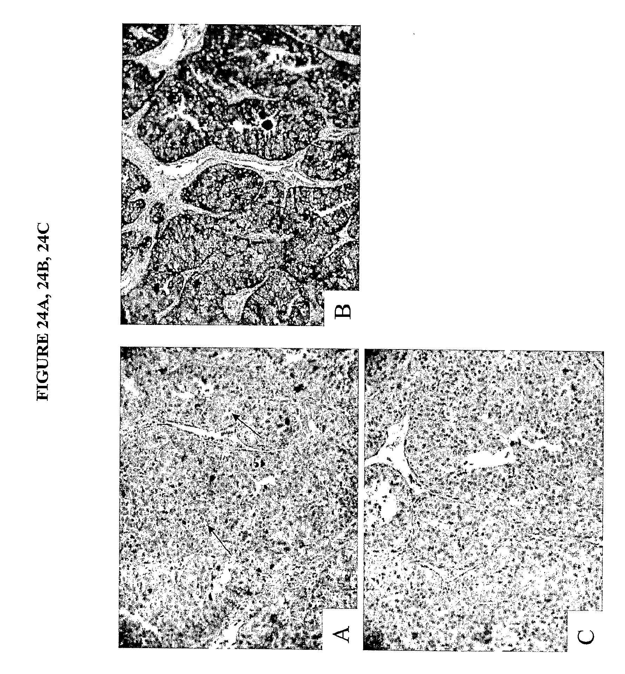

As outlined in Table 8, 7BD-33-11A stained a number of various human cancers besides breast. The following tumor types were positive for 7BD-33-11A: skin (1/2), lung (3/4), liver (2/3), stomach (4/5), thyroid (2/2), prostate (1/1), uterus (4/4) and kidney (3/3) (Figure 24A). Several other tumor types also occasionally stained positive. Other tumor tissues were negative for 7BD-33-11A expression; ovary (0/3), testis (0/1), brain (0/2) and lymph node (0/2). Conversely, 1A245.6 stained every tumor tissue type tested. However, some of the strongest staining was seen on malignant cells of the skin, lung, liver, uterus, kidney (Figure 24B), stomach and bladder. As seen with the breast cancers, 7BD-33-11A and 1A245.6 staining was localized on the membrane and within the cytoplasm of cancerous cells.

Therefore, it appears that the 7BD-33-11A and 1A245.6 antigen is not solely found on the membranes of breast cancers but also on the membrane of a large variety of tumor types including prostate. These results indicate that both 7BD-33-11A and 1A245.6 have potential as a therapeutic drug in a wide variety of tumor types in addition to breast and prostate cancer.

**Table 8: IHC On Human Multi-Tumor Tissue Array**

| Sec.No. | Age | Sex | Organ | Diagnosis | 1A245.6 | 7BD-33-11A |
|---|---|---|---|---|---|---|
| 1 | 59 | M | Skin | Malignant melanoma | ++ M/C | ++M/C |
| 2 | 25 | F | Skin | SSC | ++ M/C | |
| 3 | 50 | F | Breast | Infiltrating ductal carcinoma | ++ M/C | ++M/C |
| 4 | 57 | F | Breast | Invasive papillary carcinoma | + M /C | |
| 5 | 35 | F | Breast | Infiltrating lobular carcinoma | + M/C | F |
| 6 | 40 | M | Lymph node | Malignant lymphoma, immunoplastic | +M/C | - |
| 7 | 58 | M | Lymph node | Metastatic adenoca from stomach | +M/C | - |
| 8 | 53 | F | Bone | Osteosarcoma | ++ M/C | ++M/C |
| 9 | 26 | M | Bone | Giant cell tumor | ++ M/C | - |
| 10 | 40 | M | Bone | Chondrosarcoma | CS | CS |
| 11 | 51 | F | So ft tissue | Liposarcoma | +/- | - |
| 12 | 47 | F | So ft tissue | Neuro fibromatosis | +/- | - |
| 13 | 74 | M | Nasalcavity | In verted papillo ma | +M/C | +/- |
| 14 | 57 | M | Larynx | SCC | + Tumor, ++ Lymphocytes, Stroma | +/- |
| 15 | 60 | M | Lung | Adenocarcinoma | +++ M/C | ++ M/C |
| 16 | 51 | F | Lung | SCC | ++ M/C | + M/C |
| 17 | 68 | F | Lung | Adenocarcinoma | +M/C | - |
| 18 | 60 | M | Lung | Small cell carcinoma | ++ M/C | + M/C |
| 19 | 88 | F | Tongue | SCC | +M/C | +/- |
| 20 | 34 | F | Parotid gland | Pleomorphic adenoma | +/- mucin | - |
| 21 | 50 | F | Parotid gland | Warthin tumor | +++ M/C | +++ M/C |
| 22 | 40 | F | Parotid gland | Pleomorphic adenoma | +M/C | +/- |
| 23 | 56 | M | Submandibulargland | Salivary duct carcinoma | +M/C | - |
| 24 | 69 | F | Liver | Cholangiocarcinoma | +++ M/C | ++ M/C |
| 25 | 51 | M | Liver | Metastatic gastric Ca. | ++ M/C | |
| 26 | 64 | M | Liver | HCC | +++ M/C | ++ M/C |
| 27 | 62 | F | Gallbladder | Adenocarcinoma | ++ M/C | +M/C |
| 28 | 64 | F | Pancreas | Adenocarcinoma | ++ M/C | ++ M/C |
| 29 | 68 | M | Esophagus | SCC | + M/C | +/- |
| 30 | 73 | M | Stomach | Adenocarcinoma (poorly differentiated) | ++ M/C | + M/C |
| 31 | 63 | M | Stomach | Adenocarcinoma (moderately differentiated) | ++ M/C | +/- |
| 32 | 59 | F | Stomach | Signet ring cell carcinoma | ++ M/C | +/- |
| 33 | 62 | M | Stomach | malignant Lymphoma | + M/C | + Blood vessels |
| 34 | 51 | M | Stomach | Borderline stromal tumor | +++ M/C | + M/C |
| 35 | 42 | M | Small Intestine | Malignant stromal tumor | ++ M/C | - |
| 36 | 52 | F | Appendix | Pseuomyxoma peritonia | - PS | - |
| 37 | 53 | M | Colon | Adenocarcinoma | +M/C | +/- |
| 38 | 67 | M | Rectum | Adenocarcinoma | ++ M/C | - |
| 39 | 75 | F | Kidney | Transitional cell carcinoma | +++ M/C | ++ M/C |
| 40 | 54 | F | Kidney | Renal cell carcinoma | ++ M/C | +/- |
| 41 | 75 | F | Kidney | Renal cell carcinoma | +++ M/C | ++ M/C |
| 42 | 65 | M | Urinary bladder | Carcinoma (poorly differentiated) | ++ M/C | - |
| 43 | 67 | M | Urinary bladder | Tran sitional cell carcinoma (high grade) | +++ M/C | +++ M/C |
| 44 | 62 | M | Prostate | A den oc arcinoma | + M/C | +/- |
| 45 | 30 | M | Testis | Semin oma | + M/C | - |
| 46 | 68 | F | Uterus | En do metrial aden ocarcinoma | +++ M/C | ++ M/C |
| 47 | 57 | F | Uterus | Leimyosacoma | + C | +/- |
| 48 | 45 | F | Uterus | Leiomyoma | ++ C | +/- |
| 49 | 63 | F | Uterine cervix | SCC | + Tumor, ++ Stroma, Lymph ocytes | +/- |
| 50 | 12 | F | Ovary | Endodermal sinus tumor | ++ M/C | - |
| 51 | 33 | F | Ovary | Mucinous adenocarcinoma | + M/C | - |
| 52 | 70 | F | Ovary | Fibrothecoma | ++ M/C | - |
| 53 | 67 | F | Adrenalgland | Cortical carcinoma | ++ M/C | + M/C |
| 54 | 61 | F | Adrenalgland | Pheohromcytoma | +++ M/C | - |
| 55 | 54 | M | Thyroid | Papillary carcinoma | +++ M/C | ++ M/C |
| 56 | 58 | F | Thyroid | Minimally invasive follicular carcinoma | ++ M/C | + M/C |
| 57 | 74 | M | Thymus | Thymoma | + C | +/- |
| 58 | 66 | F | Brain | Meningioma | ++ M/C | - |
| 59 | 62 | M | Brain | Glioblastoma multiforme | + M/C | - |

| | | | | | | |
|---|---|---|---|---|---|---|
| Abbreviations: M: Membrane staining; C: Cytoplasmic staining; M/C: Membrane-cytoplasmic staining; CS: the section is completely sloughed; PS: the section is partially sloughed; F: The section is folded; SSC: Squamous cell carcinoma; HCC: Hepatocellular carcinoma. | | | | | | |

### EXAMPLE 11

### In Vivo MCF-7 Preventative Survival Tumor Experiment

With reference to Figures 7 and 8, 4 to 8 week old female SCID mice were implanted with 5 million MCF-7 human breast cancer cells in 100 microlitres saline injected subcutaneously in the scruff of the neck. The mice were randomly divided into 2 treatment groups of 11-13 mice. On the day after implantation, 20 mg/kg of either 11BD-2E11-2 test antibody or isotype control antibody (known not to bind MCF-7 or OVCAR-3 cells) was administered intraperitoneally at a volume of 300 microliters after dilution from the stock concentration with a diluent that contained 2.7 mM KCl, 1 mM KH₂PO₄, 137 mM NaCl and 20 mM Na₂HPO₄. The antibodies were then administered once per week for a period of 7 weeks in the same fashion. Tumor growth was measured about every seventh day with calipers for up to 8 weeks or until individual animals reached the Canadian Council for Animal Care (CCAC) end-points. Body weights of the animals were recorded for the duration of the study. At the end of the study all animals were euthanised according to CCAC guidelines.

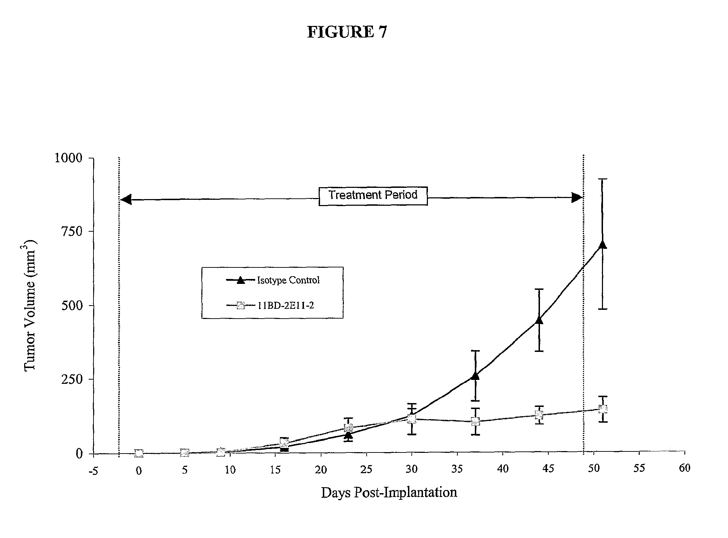

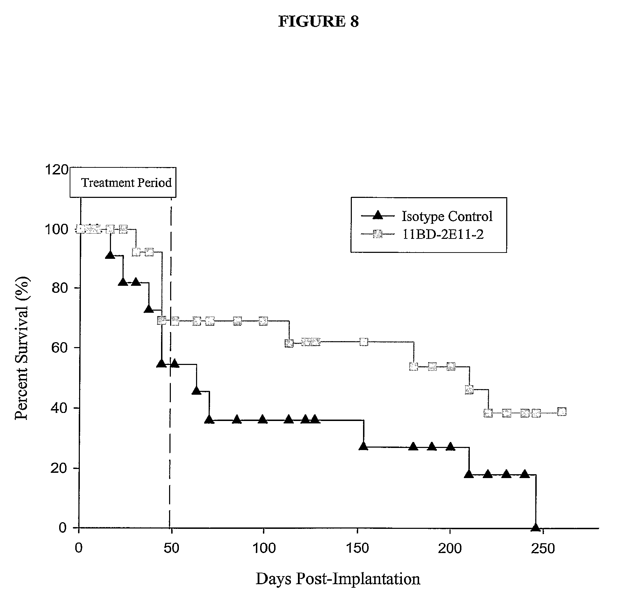

11BD-2E11-2 significantly reduced the tumor burden in treated mice in comparison to controls (Figure 7). After treatment (day 51), 11BD-2E11-2 prevented tumor growth by 80 percent (p=0.0098) in comparison to isotype control antibody treated mice. There was also a post-treatment survival benefit (Figure 8) associated with 11BD-2E11-2 administration. The isotype control antibody treated group reached 100 percent mortality by day 197 post-treatment while 40 percent of the 11BD-2E11-2 treated group were still alive at day 233. In summary, 11BD-2E11-2 increased survival and decreased tumor burden in a well-established model of human breast cancer (Blakey et al. Clinical Cancer Research 8:1974-1983 2002; Klement et al. Clinical Cancer Research 8:221-232 2002; Waud et al. Relevance of Tumor Models for Anticancer Drug Development, Fiebig and Burger, eds. 54:305-315 1999; Karpanen et al. Cancer Research 61:1786-1790 2001).

### EXAMPLE 12

### In Vivo OVCAR-3 Preventative Tumor Experiments

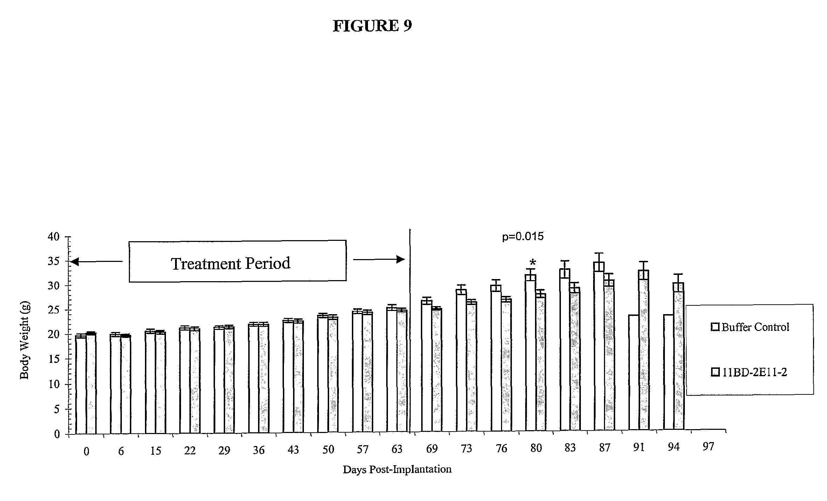

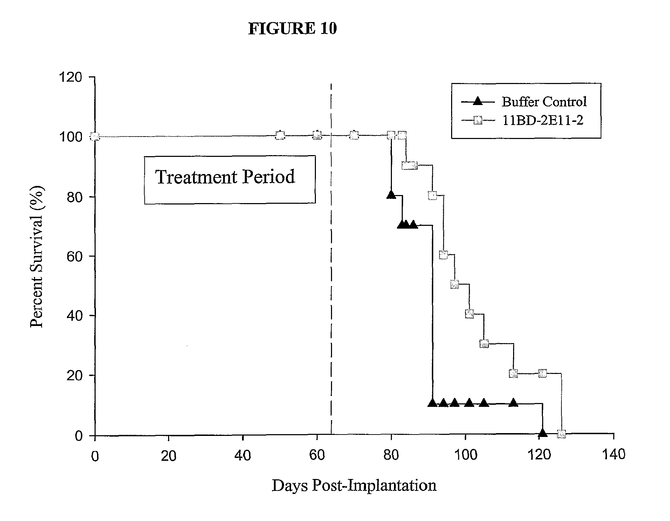

With reference to the data shown in Figures 9 and 10, 4 to 8 week old, female SCID mice were implanted with 5 million OVCAR-3 human ovarian cancer cells in 1000 microliters saline injected intraperitoneally. The mice were randomly divided into 2 treatment groups of 10. On the day after implantation, 20 mg/kg of 11BD-2E11-2 test antibody or buffer control antibody was administered intraperitoneally at a volume of 300 microliters after dilution from the stock concentration with a diluent that contained 2.7 mM KCl, 1 mM KH₂PO₄, 137 mM NaCl and 20 mM Na₂HPO₄. The antibodies were then administered once per week for a period of 9 weeks in the same fashion. Body weights of the animals were recorded for the duration of the study. At the end of the study all animals were euthanised according to CCAC guidelines.
In the OVCAR-3 ovarian cancer xenograft model, increasing body weight can be used increasing body weight can be used as a surrogate indicator of disease progression since this reflects the accumulation of ascites from increased tumor burden (Figure 9). At day 80 post-implantation (16 days after the end of treatment), 11BD-2E11-2 administration prevented body weight gain by 12.4 percent (p=0.015) compared to the buffer control group. Mice were monitored post-treatment for survival (Figure 10). By day 87, the buffer control group had reached 90 percent mortality while the 11BD-2E11-2 treated group still had 80 percent survival. The 11BD-2E11-2 treated group did not reach 90 percent mortality until day 125. In summary, 11BD-2E11-2 antibody treatment reduced tumor burden, delayed disease progression and enhanced survival in comparison to a buffer control antibody in a well-recognized model of human ovarian cancer. Therefore treatment with 11BD-2E11-2 significantly decreased the tumor burden of established tumors in two well-recognized models of human cancer disease (breast and ovarian cancers) suggesting pharmacologic and pharmaceutical benefits of this antibody for therapy in other mammals, including man (Smith et al. The Prostate 48:47-53 2001; Olson et al. International Journal of Cancer 98:923-929 2002; Guilbaud et al. Clinical Cancer Research 7:2573-2580 2001; Von Gruenigen et al. International Journal of Gynecologic Cancer 9:365-372 1999; Guichard et al. Clinical Cancer Research 7:3222-3228 2001; Xiao et al. Protein Expression and Purification 19:12-21 2000).
All patents and publications mentioned in this specification are indicative of the levels of those skilled in the art to which the invention pertains. All patents and publications are herein incorporated by reference to the same extent as if each individual publication was specifically and individually indicated to be incorporated by reference.

It is to be understood that while a certain form of the invention is illustrated, it is not to be limited to the specific form or arrangement of parts herein described and shown. It will be apparent to those skilled in the art that various changes may be made without departing from the scope of the invention and the invention is not to be considered limited to what is shown and described in the specification.

One skilled in the art will readily appreciate that the present invention is well adapted to carry out the objects and obtain the ends and advantages mentioned, as well as those inherent therein. Any oligonucleotides, peptides, polypeptides, biologically related compounds, methods, procedures and techniques described herein are presently representative of the preferred embodiments, are intended to be exemplary and are not intended as limitations on the scope. Changes therein and other uses will occur to those skilled in the art which are encompassed within the spirit of the invention and are defined by the scope of the appended claims. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

The invention also includes the subject matter of the claims of WO2004/065422 from which this application is derived, the content of which is reproduced below.
Paragraph 1. A method of extending survival and/or delaying disease progression by treating a human tumor in a mammal, wherein said tumor expresses an antigen which specifically binds to a monoclonal antibody or antigen binding fragment thereof which has the identifying characteristics of a monoclonal antibody encoded by a clone deposited with the ATCC as accession number PTA-4890, comprising administering to said mammal said monoclonal antibody in an amount effective to reduce said mammal's tumor burden, whereby disease progression is delayed and/or survival is extended.
Paragraph 2. The method of paragraph 1 wherein said antibody is conjugated to a cytotoxic moiety.
Paragraph 3. The method of paragraph 2 wherein said cytotoxic moiety is a radioactive isotope.
Paragraph 4. The method of paragraph 1 wherein said antibody activates complement.
Paragraph 5. The method of paragraph 1 wherein said antibody mediates antibody dependent cellular cytotoxicity.
Paragraph 6. The method of paragraph 1 wherein said antibody is a murine antibody.
Paragraph 7. The method of paragraph 1 wherein said antibody is a humanized antibody
Paragraph 8. The method of paragraph 1 wherein said antibody is a chimerized antibody.
Paragraph 9. An isolated monoclonal antibody or antigen binding fragments thereof encoded by the clone deposited with the ATCC as accession number PTA-4890.
Paragraph 10. The isolated antibody or antigen binding fragments of paragraph 9, wherein said isolated antibody or antigen binding fragments thereof is humanized.
Paragraph 11. The isolated antibody or antigen binding fragments of paragraph 9 conjugated with a member selected from the group consisting of cytotoxic moieties, enzymes, radioactive compounds, and hematogenous cells.
Paragraph 12. The isolated antibody or antigen binding fragments of paragraph 9, wherein said isolated antibody or antigen binding fragments thereof is a chimerized antibody.
Paragraph 13. The isolated antibody or antigen binding fragments of paragraph 9, wherein said isolated antibody or antigen binding fragments thereof is a murine antibody.
Paragraph 14. The isolated clone deposited with the ATCC as accession number PTA-4890.
Paragraph 15. A binding assay to determine presence of cancerous cells in a tissue sample selected from a human tumor comprising:
   providing a tissue sample from said human tumor ;
   providing an isolated monoclonal antibody or antigen binding fragment thereof encoded by the clone deposited with the ATCC as accession number PTA-4890;
   contacting said isolated monoclonal antibody or antigen binding fragment thereof with said tissue sample; and
   determining binding of said isolated monoclonal antibody or antigen binding fragment thereof with said tissue sample;
   whereby the presence of said cancerous cells in said tissue sample is indicated.
Paragraph 16. The binding assay of paragraph 15 wherein the human tumor tissue sample is obtained from a tumor originating in a tissue selected from the group consisting of colon, ovarian, lung, and breast tissue.
Paragraph 17. A process of isolating or screening for cancerous cells in a tissue sample selected from a human tumor comprising:
   providing a tissue sample from a said human tumor ;
   providing an isolated monoclonal antibody or antigen binding fragment thereof encoded by the clone deposited with the ATCC as accession number PTA-4890;
   contacting said isolated monoclonal antibody or antigen binding fragment thereof with said tissue sample; and
   determining binding of said isolated monoclonal antibody or antigen binding fragment thereof with said tissue sample;
   whereby said cancerous cells are isolated by said binding and their presence in said tissue sample is confirmed.
Paragraph 18. The process of paragraph 17 wherein the human tumor tissue sample is obtained from a tumor originating in a tissue selected from the group consisting of colon, ovarian, lung, and breast tissue.
Paragraph 19. A method of extending survival and/or delaying disease progression by treating a human tumor in a mammal, wherein said tumor expresses an antigen which specifically binds to a monoclonal antibody or antigen binding fragment thereof which has the identifying characteristics of a monoclonal antibody encoded by a clone deposited with the ATCC as accession number PTA-4889, comprising administering to said mammal said monoclonal antibody in an amount effective to reduce said mammal's tumor burden, whereby disease progression is delayed and/or survival is extended.
Paragraph 20. The method of paragraph 19 wherein said antibody is conjugated to a cytotoxic moiety.
Paragraph 21. The method of paragraph 20 wherein said cytotoxic moiety is a radioactive isotope.
Paragraph 22. The method of paragraph 19 wherein said antibody activates complement.
Paragraph 23. The method of paragraph 19 wherein said antibody mediates antibody dependent cellular cytotoxicity.
Paragraph 24. The method of paragraph 19 wherein said antibody is a murine antibody.
Paragraph 25. The method of paragraph 19 wherein said antibody is a humanized antibody
Paragraph 26. The method of paragraph 19 wherein said antibody is a chimerized antibody.
Paragraph 27. An isolated monoclonal antibody or antigen binding fragments thereof encoded by the clone deposited with the ATCC as accession number PTA-4889.
Paragraph 28. The isolated antibody or antigen binding fragments of paragraph 27, wherein said isolated antibody or antigen binding fragments thereof is humanized.
Paragraph 29. The isolated antibody or antigen binding fragments of paragraph 27 conjugated with a member selected from the group consisting of cytotoxic moieties, enzymes, radioactive compounds, and hematogenous cells.
Paragraph 30. The isolated antibody or antigen binding fragments of paragraph 27, wherein said isolated antibody or antigen binding fragments thereof is a chimerized antibody.
Paragraph 31. The isolated antibody or antigen binding fragments of paragraph 27, wherein said isolated antibody or antigen binding fragments thereof is a murine antibody.
Paragraph 32. The isolated clone deposited with the ATCC as accession number PTA-4889.
Paragraph 33. A binding assay to determine presence of cancerous cells in a tissue sample selected from a human tumor comprising:
   providing a tissue sample from said human tumor ;
   providing an isolated monoclonal antibody or antigen binding fragment thereof encoded by the clone deposited with the ATCC as accession number PTA-4889;
   contacting said isolated monoclonal antibody or antigen binding fragment thereof with said tissue sample; and
   determining binding of said isolated monoclonal antibody or antigen binding fragment thereof with said tissue sample;
   whereby the presence of said cancerous cells in said tissue sample is indicated.
Paragraph 34. The binding assay of paragraph 33 wherein the human tumor tissue sample is obtained from a tumor originating in a tissue selected from the group consisting of colon, ovarian, lung, and breast tissue.
Paragraph 35. A process of isolating or screening for cancerous cells in a tissue sample selected from a human tumor comprising:
   providing a tissue sample from a said human tumor ;
   providing an isolated monoclonal antibody or antigen binding fragment thereof encoded by the clone deposited with the ATCC as accession number PTA-4889:
   contacting said isolated monoclonal antibody or antigen binding fragment thereof with said tissue sample; and
   determining binding of said isolated monoclonal antibody or antigen binding fragment thereof with said tissue sample;
   whereby said cancerous cells are isolated by said binding and their presence in said tissue sample is confirmed.
Paragraph 36. The process of paragraph 35 wherein the human tumor tissue sample is obtained from a tumor originating in a tissue selected from the group consisting of colon, ovarian, lung, and breast tissue.
Paragraph 37. A method of extending survival and/or delaying disease progression by treating a human tumor in a mammal, wherein said tumor expresses an antigen which specifically binds to a monoclonal antibody or antigen binding fragment thereof which has the identifying characteristics of a monoclonal antibody encoded by a clone deposited with the ATCC as accession number PTA-5643, comprising administering to said mammal said monoclonal antibody in an amount effective to reduce said mammal's tumor burden, whereby disease progression is delayed and/or survival is extended.
Paragraph 38. The method of paragraph 37 wherein said antibody is conjugated to a cytotoxic moiety.
Paragraph 39. The method of paragraph 38 wherein said cytotoxic moiety is a radioactive isotope.
Paragraph 40. The method of paragraph 37 wherein said antibody activates complement.
Paragraph 41. The method of paragraph 37 wherein said antibody mediates antibody dependent cellular cytotoxicity.
Paragraph 42. The method of paragraph 37 wherein said antibody is a murine antibody.
Paragraph 43. The method of paragraph 37 wherein said antibody is a humanized antibody.
Paragraph 44. The method of paragraph 37 wherein said antibody is a chimerized antibody.
Paragraph 45. An isolated monoclonal antibody or antigen binding fragments thereof encoded by the clone deposited with the ATCC as accession number PTA-5643.
Paragraph 46. The isolated antibody or antigen binding fragments of paragraph 45, wherein said isolated antibody or antigen binding fragments thereof is humanized.
Paragraph 47. The isolated antibody or antigen binding fragments of paragraph 45 conjugated with a member selected from the group consisting of cytotoxic moieties, enzymes, radioactive compounds, and hematogenous cells.
Paragraph 48. The isolated antibody or antigen binding fragments of paragraph 45, wherein said isolated antibody or antigen binding fragments thereof is a chimerized antibody.
Paragraph 49. The isolated antibody or antigen binding fragments of paragraph 45, wherein said isolated antibody or antigen binding fragments thereof is a murine antibody.
Paragraph 50. The isolated clone deposited with the ATCC as accession number PTA-5643.
Paragraph 51. A binding assay to determine presence of cancerous cells in a tissue sample selected from a human tumor comprising:
   providing a tissue sample from said human tumor ;
   providing an isolated monoclonal antibody or antigen binding fragment thereof encoded by the clone deposited with the ATCC as accession number PTA-5643;
   contacting said isolated monoclonal antibody or antigen binding fragment thereof with said tissue sample; and
   determining binding of said isolated monoclonal antibody or antigen binding fragment thereof with said tissue sample;
   whereby the presence of said cancerous cells in said tissue sample is indicated.
Paragraph 52. The binding assay of paragraph 51 wherein the human tumor tissue sample is obtained from a tumor originating in a tissue selected from the group consisting of colon, ovarian, lung, and breast tissue.
Paragraph 53. A process of isolating or screening for cancerous cells in a tissue sample selected from a human tumor comprising:
   providing a tissue sample from a said human tumor ;
   providing an isolated monoclonal antibody or antigen binding fragment thereof encoded by the clone deposited with the ATCC as accession number PTA-5643:
   contacting said isolated monoclonal antibody or antigen binding fragment thereof with said tissue sample; and
   determining binding of said isolated monoclonal antibody or antigen binding fragment thereof with said tissue sample;
   whereby said cancerous cells are isolated by said binding and their presence in said tissue sample is confirmed.
Paragraph 54. The process of paragraph 53 wherein the human tumor tissue sample is obtained from a tumor originating in a tissue selected from the group consisting of colon, ovarian, lung, and breast tissue.

## Claims

1. A monoclonal antibody or antigen binding fragment thereof which has the identifying characteristics of a monoclonal antibody encoded by a clone deposited with the ATCC as accession number PTA-5643 for use in extending survival and/or delaying disease progression by treating a human tumor in a mammal, wherein said tumor expresses an antigen which specifically binds to a monoclonal antibody or antigen binding fragment thereof which has the identifying characteristics of a monoclonal antibody encoded by a clone deposited with the ATCC as accession number PTA-5643.

2. The monoclonal antibody or antigen binding fragment thereof claim 1 wherein said antibody is conjugated to a cytotoxic moiety.

3. The monoclonal antibody or antigen binding fragment thereof of claim 2 wherein said cytotoxic moiety is a radioactive isotope.

4. The monoclonal antibody or antigen binding fragment thereof of claim 31 wherein said antibody activates complement, or wherein said antibody mediates antibody dependent cellular cytotoxicity.

5. The monoclonal antibody or antigen binding fragment thereof of claim 1 wherein said antibody is a murine antibody, or wherein said antibody is a humanized antibody, or wherein said antibody is a chimerized antibody.

6. An isolated monoclonal antibody or antigen binding fragments thereof encoded by the clone deposited with the ATCC as accession number PTA-5643.

7. The isolated antibody or antigen binding fragments of claim 6, wherein said isolated antibody or antigen binding fragments thereof is humanized.

8. The isolated antibody or antigen binding fragments of claim 6 conjugated with a member selected from the group consisting of cytotoxic moieties, enzymes, radioactive compounds, and hematogenous cells.

9. The isolated antibody or antigen binding fragments of claim 6, wherein said isolated antibody or antigen binding fragments thereof is a chimerized antibody.

10. The isolated antibody or antigen binding fragments of claim 6, wherein said isolated antibody or antigen binding fragments thereof is a murine antibody.

11. The isolated clone deposited with the ATCC as accession number PTA-5643.

12. A binding assay to determine presence of cancerous cells in a tissue sample selected from a human tumor comprising:
providing a tissue sample from said human tumor ;
providing an isolated monoclonal antibody or antigen binding fragment thereof encoded by the clone deposited with the ATCC as accession number PTA-5643;
contacting said isolated monoclonal antibody or antigen binding fragment thereof with said tissue sample; and
determining binding of said isolated monoclonal antibody or antigen binding fragment thereof with said tissue sample;
whereby the presence of said cancerous cells in said tissue sample is indicated.

13. A process of isolating or screening for cancerous cells in a tissue sample selected from a human tumor comprising:
providing a tissue sample from a said human tumor ;
providing an isolated monoclonal antibody or antigen binding fragment thereof encoded by the clone deposited with the ATCC as accession number PTA-5643:
contacting said isolated monoclonal antibody or antigen binding fragment thereof with said tissue sample; and
determining binding of said isolated monoclonal antibody or antigen binding fragment thereof with said tissue sample;
whereby said cancerous cells are isolated by said binding and their presence in said tissue sample is confirmed.

14. The assay of claim 12 or the process of claim 13 wherein the human tumor tissue sample is obtained from a tumor originating in a tissue selected from the group consisting of colon, ovarian, lung, and breast tissue.

15. Use of an isolated monoclonal antibody encoded by a clone deposited with the ATCC as accession number PTA-5643 or an antigen binding fragment thereof in the manufacture of a medicament for treating cancer.
